# EUROPÄISCHE PATENTSCHRIFT

(11) **EP 1 598 421 B1**
(45) Veröffentlichungstag und Bekanntmachung des Hinweises auf die Patenterteilung: **26.01.2011**
(21) Anmeldenummer: 05103692.9
(22) Anmeldetag: 03.05.2005
(51) Int. Cl.: C12N 15/11

(54) **Oligoribonukleotide zur Beeinflussung des Haarwachstums**
Oligoribonucleotides for influencing hair growth
Oligoribonucleotides pour influencer la pousse des cheveux

(30) Priorität: 19.05.2004 DE 102004025881
(43) Veröffentlichungstag der Anmeldung: 23.11.2005
(73) Patentinhaber: Beiersdorf AG, 20245 Hamburg (DE)
(72) Erfinder: Breitenbach, Ute, 20251 Hamburg (DE); Saenger, Kyra, 22081 Hamburg (DE); Schmidt-Rose, Thomas, 22529 Hamburg (DE)

(56) Entgegenhaltungen:
- WO-A-94/25588
- SPEARMAN M ET AL: "Antisense oligodeoxyribonucleotide inhibition of TGF-beta1 gene expression and alterations in the growth and malignant properties of mouse fibrosarcoma cells" GENE, ELSEVIER BIOMEDICAL PRESS. AMSTERDAM, NL, Bd. 149, 1994, Seiten 25-29, XP002089439 ISSN: 0378-1119
- "Inhibition of TGF-B1 exprssion by antisense RNA suppressed extracellular matrix accumulation in experimental liver fibrosis - Zhiqing Liang Sr., Yong Chen, Changxu Shi, Southwest Hospital, Chongqing China" HEPATOLOGY, WILLIAMS AND WILKINS, BALTIMORE, MD, US, Bd. 34, Nr. 4, Oktober 2001 (2001-10), Seite A396, XP004716428 ISSN: 0270-9139
- INUI S. ET AL.,: "Identification of androgen-inducible TGF-b1 derived from dermal papilla cells as a key mediator in androgenetic alopecia" J. INVESTIGATIVE DERMATOLOGY SYMPOSIUM PROCEEDINGS, Bd. 8, Nr. 1, 2003, Seiten 69-71, XP002462202
- WELKER P. ET AL.,: "hair cycle-dependent changes in the gene expression and protein content of transforming factor beta 1 and beta 3 in murine skin." ARCH. DERMATOL. RES., Bd. 289, 1997, Seiten 554-557, XP002462203

<110> Beiersdorf AG
<120> oligoribonukleotide zur Beeinflussung des Haarwachstums
<130> Prtpl-02073
<160> 46
<170> Patentin version 3.1
<210> 1
   <211> 19
   <212> DNA
   <213> Homo sapiens
<400> 1
   cagcacccgc gaccgggtg 19
<210> 2
   <211> 19
   <212> DNA
   <213> Homo sapiens
<400> 2
   cacatcagag ctccgagaa 19
<210> 3
   <211> 19
   <212> DNA
   <213> Homo sapiens
<400> 3
   aagtggagca gcacgtgga 19
<210> 4
   <211> 19
   <212> DNA
   <213> Homo sapiens
<400> 4
   attgagggct ttcgcctta 19
<210> 5
   <211> 19
   <212> DNA
   <213> Homo sapiens
<400> 5
   ccagcataac ccgggcgcc 19
<210> 6
   <211> 19
   <212> DNA
   <213> Homo sapiens
<400> 6
   gcccaaggtg gagcagctg 19
<210> 7
   <211> 19
   <212> DNA
   <213> Homo sapiens
<400> 7
   cagcacccgc gaccgggtg 19
<210> 8
   <211> 19
   <212> DNA
   <213> Homo sapiens
<400> 8
   cacatcagag ctccgagaa 19
<210> 9
   <211> 19
   <212> DNA
   <213> Homo sapiens
<400> 9
   aagtggagca gcacgtgga 19
<210> 10
   <211> 19
   <212> DNA
   <213> Homo sapiens
<400> 10
   attgagggct ttcgcctta 19
<210> 11
   <211> 19
   <212> DNA
   <213> Homo sapiens
<400> 11
   ccagcataac ccgggcgcc 19
<210> 12
   <211> 19
   <212> DNA
   <213> Homo sapiens
<400> 12
   gcccaaggtg gagcagctg 19
<210> 13
   <211> 19
   <212> DNA
   <213> Homo sapiens
<400> 13
   gcagtttatc cccaatgtg 19
<210> 14
   <211> 19
   <212> ONA
   <213> Homo sapiens
<400> 14
   ggatgaagaa aacaccgga 19
<210> 15
   <211> 4
   <212> DNA
   <213> Homo sapiens
<400> 15
   ggac 4
<210> 16
   <211> 19
   <212> DNA
   <213> Homo sapiens
<400> 16
   gttgaacgct ttggccatc 19
<210> 17
   <211> 19
   <212> DNA
   <213> Homo sapiens
<400> 17
   agcagagaac tcggtggcg 19
<210> 18
   <211> 4
   <212> DNA
   <213> Homo sapiens
<400> 18
   cgcc 4
<210> 19
   <211> 19
   <212> DNA
   <213> Homo sapiens
<400> 19
   ggagttgtgt aaggcagtg 19
<210> 20
   <211> 19
   <212> DNA
   <213> Homo sapiens
<400> 20
   ggttctctgc tagacgaca 19
<210> 21
   <211> 19
   <212> DNA
   <213> Homo sapiens
<400> 21
   ctgccgtcta ccctgtctc 19
<210> 22
   <211> 19
   <212> DNA
   <213> Homo sapiens
<400> 22
   gtccggagca ctggacgag 19
<210> 23
   <211> 19
   <212> DNA
   <213> Homo sapiens
<400> 23
   cccgctggac tacggcagc 19
<210> 24
   <211> 19
   <212> DNA
   <213> Homo sapiens
<400> 24
   atgggcccct ggatggata 19
<210> 25
   <211> 19
   <212> DNA
   <213> Homo sapiens
<400> 25
   ggtcttcttc aaaagagcc 19
<210> 26
   <211> 4
   <212> DNA
   <213> Homo sapiens
<400> 26
   ggga 4
<210> 27
   <211> 19
   <212> DNA
   <213> Homo sapiens
<400> 27
   cagaagtacc tgtgcgcca 19
<210> 28
   <211> 19
   <212> DNA
   <213> Homo sapiens
<400> 28
   tgattgcact attgataaa 19
<210> 29
   <211> 3
   <212> DNA
   <213> Homo sapiens
<400> 29
   gga 3
<210> 30
   <211> 19
   <212> DNA
   <213> Homo sapiens
<400> 30
   aattgtccat cttgtcgtc 19
<210> 31
   <211> 19
   <212> DNA
   <213> Homo sapiens
<400> 31
   actacaggag gaaggagag 19
<210> 32
   <211> 19
   <212> DNA
   <213> Homo sapiens
<400> 32
   ggctatgaat gtcagccca 19
<210> 33
   <211> 19
   <212> DNA
   <213> Homo sapiens
<400> 33
   ccagcccgac tcctttgca 19
<210> 34
   <211> 19
   <212> DNA
   <213> Homo sapiens
<400> 34
   ctccaggatg ctctacttc 19
<210> 35
   <211> 19
   <212> DNA
   <213> Homo sapiens
<400> 35
   tgagtaccgc atgcacaag 19
<210> 36
   <211> 19
   <212> DNA
   <213> Homo sapiens
<400> 36
   gagtttggat ggctccaaa 19
<210> 37
   <211> 19
   <212> DNA
   <213> Homo sapiens
<400> 37
   gcactgctac tcttcagca 19
<210> 38
   <211> 2
   <212> DNA
   <213> Homo sapiens
<400> 38
   99 2
<210> 39
   <211> 19
   <212> DMA
   <113> Homo sapiens
<400> 39
   ctcgatcgta tcattgcat 19
<210> 40
   <211> 19
   <212> DNA
   <213> Homo sapiens
<400> 40
   agaaaaaatc ccacatcct 19 <210> 41
   <211> 19
   <212> DNA
   <213> Homo sapiens
<400> 41
   gtgctgcttt tgtgatttc 19
<210> 42
   <211> 4
   <212> DNA
   <213> Homo sapiens
<400> 42
   ggtg 4
<210> 43
   <211> 19
   <212> DNA
   <213> Homo sapiens
<400> 43
   gatgcacacc atgtcctcc 19
<210> 44
   <211> 19
   <212> DNA
   <213> Homo sapiens
<400> 44
   gcctgccaag tcagctcgc 19
<210> 45
   <211> 7
   <212> DNA
   <213> Homo sapiens
<400> 45
   gacccag 7
<210> 46
   <211> 19
   <212> DNA
   <213> Homo sapiens
<400> 46
   ggaagtacat ttgaagaac 19

## Beschreibung

Die vorliegende Erfindung betrifft die Verwendung von Oligoribonukleotiden, die den Abbau der mRNA von Proteinen und Enzymen induzieren, die in stimulierender Art und Weise auf den Haarzyklus Einfluss nehmen zur Herstellung kosmetischer oder dermatologischer Zubereitungen für die Behandlung und Prophylaxe des verminderten (Haarausfall, Haarminiaturisierung) Wachstums von Haaren der Kopfhaut oder des Körpers, wie sie beispielsweise mit altersbedingen Haarwuchsveränderungen einhergehen.

Unter den Haarzyklus regulierenden Proteinen oder Enzymen werden in erster Linie Haarwuchs inhibierende Proteine wie Wachstumsfaktoren, insbesondere die Mitglieder der Tumor Growth Factor beta-Superfamilie (TGFbeta) und deren Rezeptoren (TGFbeta-R), und Steroidrezeptoren und Steroid katalysierende Enzyme, insbesondere der Androgen-Rezeptor, sowie Haarwuchs fördernde zelluläre Signalmoleküle, insbesondere das Sonic Hedgehog Protein und der Insulin ähnliche Wachstumsfaktor 1 (IGF-1), verstanden.

Papillenhaar wird das Haar genannt, das sich in der Wachstumsperiode befindet, die auch anagene Phase oder Anagenphase genannt wird. In dieser Phase ist das Haar mit seiner Papille in der Haut verankert. Etwa 80% der Kopfhaare befinden sich etwa 2 bis 5 Jahre lang in der Anagenphase. In einer sich anschließenden Übergangsphase (Katagenphase) wandert das Haar etwa 2 Wochen lang an die Hautoberfläche und bleibt dann etwa 3 bis 4 Monate lang in einem Ruhestadium (Telogenphase) bis es schließlich ausfällt.

Ein über das normale Maß hinausgehender Haarausfall oder ein übermäßig starker Haarwuchs an unerwünschten Körperstellen wird zumeist als schwere kosmetische Störung angesehen, ebenso wie andere Wachstumsstörungen der Haare. Daher wurden schon viele Mittel beispielsweise zur Behandlung von Haarausfall und Glatzenbildung sowie Haarwuchsmittel vorgeschlagen, die das Wachstum der Haare erhalten oder fördern sollen.

Das Wachstum des Haarfollikels unterliegt einer cyclischen Steuerung, Wachstumsphasen (Anagen) wechseln sich mit Ruhephasen (Telogen) ab, der Übergang zwischen diesen Phasen wird als Katagen bezeichnet. Von der Länge der Anagen-Phase (i.d.R. 2 - 5 Jahre) hängen sowohl die Größe eines Haarfollikels als auch die Länge seines Haarschaftes ab (Paus el al., 1998). Eine Veränderung des normalen Haarzyklus, insbesondere eine Verkürzung der Wachstumsphase durch verfrühten Übergang in das Katagen/ Telogen, führt über mehrere Zyklen zur Rückbildung des Skalphaares und damit verbunden zu einer drastischen Veränderung seiner biophysikalischen Eigenschaften.

Das Haarwachstum wird von vielen endogenen und exogenen Faktoren bestimmt, die es hemmen oder fördem können. In diesem Zusammenhang ist bekannt, dass die Aktivität des Tumor Growth Factors beta 1 (TGFbeta-1) entscheidend für die Dauer der Wachstumsphase des Haarfollikels ist, indem TGFbeta-1 den Anagen-Katagen-Übergang fördert und die Haar-Neubildung unterdrückt (Foitzik et al., 2000; Liu et al., 2001). Der Haarwuchs fördernde Effekt kann zudem durch die Inhibition des Androgen-Rezeptors erreicht werden. Dieses Protein wird im Haarfollikel exprimiert und ist verantwortlich für die Sensitivität der follikulären Zellen gegenüber androgenen Hormonen. Diese Hormone sind bekanntermaßen für die Verkürzung der Anagenphase verantwotlich und bedingen bei außreichender Sensitivität der Haarfollikel einen verschlechterten Haarwuchs (Hibberts et al., 1998). Wirkstoffe, die die Synthese von TGFbeta-1 und/ oder des Androgen-Rezeptors hemmen, führen daher zu einer verbesserten Haarbildung (Haarkräftigung) und sind somit von grossem kosmetischen Nutzen.

Auf der anderen Seite bietet die Inhibition der Translation von Genen, die die Haarbildung fördern (z.B. das Sonic Hedgehog Gen, Sato et al. (1999); oder das Gen des Insulin ähnlichen Wachstumsfaktors 1, Su et al. (1999)), eine Möglichkeit, unerwünschten Haarwuchs zu unterdrücken. Eine Inhibition der zellulären Sonic Hedgehog-Synthese beschleunigt den Anagen-Telogen Transit, so dass der Haarfollikel in die Ruhephase übergeht. Ergebnis ist eine verkürzte Wuchsphase und ein dünneres, weniger sichtbares Haar. Dieser Ansatz ist besonders für die Anwendung im Shaving- oder Body-Bereich sinnvoll, um unerwünschte Körperbehaarung zu reduzieren.

### Näheres dazu findet sich in

Paus, R. (1998) Principles of hair cycle control. J Dermatol. 25(12):793-802,

Foitzik K, Lindner G, Mueller-Roever S, Maurer M, Botchkareva N, Botchkarev V, Handjiski B, Metz M, Hibino T, Soma T, Dotto GP, Paus R (2000): Control of murine hair follicle regression (catagen) by TGF-beta1 in vivo. FASEB J. 14 (5): 752-60,

Liu X, Alexander V, Vijayachandra K, Bhogte E, Diamond I, Glick A (2001): Conditional epidermal expression of TGFbeta 1 blocks neonatal lethality but causes a reversible hyperplasia and alopecia. Proc Natl Acad Sci U S A 98 (16): 9139-44,

Hibberts, N. A., A. E. Howell and Randall, V. A. (1998). "Balding hair follicle dermal papilla cells contain higher levels of androgen receptors than those from non-balding scalp." J Endocrinol 156(1): 59-65.,

Sato, N., P. L. Leopold and Crystal, R. G. (1999). "Induction of the hair growth phase in postnatal mice by localized transient expression of Sonic hedgehog." J Clin Invest 104(7): 855-864 und

Su, H. Y., J. G. Hickford, Bickerstaffe, R. and Palmer, B. R. (1999). "Insulin-like growth factor 1 and hair growth." Dermatol Online J 5(2): 1.

Fire et al., Trends Genet. 15 (1999) 358-363 haben gezeigt, dass sich die Genexpression posttranskriptional durch die Anwesenheit doppelsträngiger RNA-Fragmente (dsRNA), die homolog zur Sequenz der mRNA des untersuchten Gens ist, inhibieren läßt, und diesen Vorgang als RNA-Interterenz (RNAi) bezeichnet. Die dsRNA bewirkt auf noch ungeklärte Weise den spezifischen Abbau der homologen mRNA in der Zelle und verhindert so die Proteinproduktion.

Die WO01/29058 offenbart die Identifikation von Genen, die an der RNAi beteiligt sind, sowie deren Verwendung zur Modulation des RNAi-Aktivität.

Elbashir et al., Nature 411 (2001) 494-498, beschreiben die spezifische Inhibierung der Expression von endogenen und heterologen Genen in verschiedenen Säugerzellen durch kurze, interferierende RNAs (short interfering RNAs, siRNAs). Es wurden doppelsträngige RNA-Fragmente mit einer Länge von 21 Nukleotiden eingesetzt.

Aus der WO01/6883 ist die Verminderung der Genexpression in Zellen durch dsRNA bekannt. Die dsRNA enthält eine Nukleotidsequenz, die unter den physiologischen Bedingungen der Zelle mit der Nukleotidsequenz zumindest eines Teils des zu inhibierenden Gens hybridisiert. Die dsRNA weist vorzugsweise eine Länge von 400 bis 800 Nukleotiden auf.

Die WO01/75164 offenbart die Verwendung von dsRNA mit einer Länge von 21 bis 23 Nukleotiden zur spezifischen Inaktivierung von Genfunktionen in Säugerzellen durch RNAi.

Brummelkamp et al., Science 296 (2002) 550-553, beschreiben ein Vektorsystem, das die Synthese von siRNAs in Säugerzellen auslösen und so die Genexpression eines Zielgens inhibieren soll.

Die EP 1 214 945 A2 offenbart die Verwendung von dsRNA mit einer Länge von 15 bis 49 Basenpaaren zur Hemmung der Expression eines vorgegebenen Zielgens in Säugerzellen. Die dsRNA kann zur Erhöhung ihrer Stabilität modifiziert sein und soll die Behandlung von Krebs, viralen Erkrankungen und Morbus Alzheimer erlauben.

Die WO02/053773 betrifft ein in vitro Verfahren zur Bestimmung des Hautstreß und der Hautalterung bei Menschen und Tieren, zur Durchführung des Verfahrens geeignete Test-Kits und Biochips sowie ein Testverfahren zum Nachweis der Wirksamkeit von kosmetischen oder pharmazeutischen Wirkstoffen gegen Hautstreß und Hautalterung.

Die WO03074654A2 offenbart die Verwendung von dsRNA als Methode oder Reagenz zur Modulation der Genexpression u.a. für therapeutische und diagnostische Anwendungen.

US5877160 offenbart eine Methode zur Verminderung der androgenvermittelten Haarausfalls durch Verringerung des Gehalts an proteingebundenem 5-alphadihydrotestosteron in Kopfhautgewebe ohne den Testosteronmetabolismus in anderen Geweben nennenswert zu beeinflussen, wobei die Zellen der Kopfhaut einer wirksamen Menge eines Oligomers ausgesetzt werden, welches mit einem Gen wechselwirkt, das einen androgenen Rezeptor, 5-alphareductase oder dessen Transkriptionsprodukt, oder eine Zielsequenz, die direkt "upstream" an die Transkriptionsstartsequenz des entsprechenden Gens anschließt und damit die Expression des androgenen Rezeptors oder der 5-alpha-Reduktase verringert oder verhindert.

WO9418835 offenbart eine Methode zur Verminderung der androgenvermittelten Haarausfalls durch Verringerung des Gehalts an proteingebundenem 5-alphadihydrotestosteron in Kopfhautgewebe ohne den Testosteronmetabolismus in anderen Geweben nennenswert zu beeinflussen, wobei die Zellen der Kopfhaut einer wirksamen Menge eines Oligomers oder von Oligomeren ausgesetzt werden, welche mit einem Gen wechselwirken, das einen androgenen Rezeptor, 5-alpha-Reduktase oder dessen Transkriptionsprodukt, oder eine Zielsequenz, die direkt "upstream" an die Transkriptionsstartsequenz des entsprechenden Gens anschließt und damit die Expression des androgenen Rezeptors oder der 5-alpha-Reduktase verringert oder verhindert.

US5994319 offenbart näher bestimmte Oligonucleotide mit einer Nucleotidsequenz, die komplementär zu mindestens einen Teil des Prä-mRNA-Transkripts oder dem Transkript gereifter mRNA humaner 5-alpha-Reduktase Typ I oder II, wobei das Oligonucleotid mit dem mRNA-Transkript hybridisieren kann.

Auch in US5556956 wird weiteres über die Behandlung von Haarausfall unter Verwendung von Antisense Oligodeoxyribonukleotiden beschrieben.

Die Schrift US20030211065A1 offenbart ein therapeutisches Mittel zur Diagnose und/oder Therapie von Haarausfall (Alopezie) und anderen Haarwuchs assoziierten Indikationen enthaltend ein therapeutisches Agens, welches die Aktivität von Comeodesmosin auf Protein- und/ oder Genebene moduliert, u.a. durch die Verwendung von Oligoribonukleotiden.

Die Schrift WO03101376A2 offenbart die topische Applikaiton von mindestens einem doppelsträgigen RNA-Oligonukleotid zur kosmethischen oder therapeutischen Behandlung; beispielsweise der Psoriasis.

Die Schrift WO03070197A2 offenbart die Herstellung und Verwendung kurzer interferierender Nukleinsäuremoleküle, beispielsweise zur Behandlung und Diagnose von Diabetes bedingter Nephropathie, die die Expression des Transforming Growth Faktorbeta Rezeptors herunter regulieren.

Die Schrift WO03074654A2 offenbart Methoden und Reagenzien zur Modulation der Genexpression für u.a. therapeutische und diagnostische Anwendungen, die kleine interferierende Nukleinsäuremoleküle (siRNA) enthalten zur Behandlung jedweder Erkrankungen oder Indikationen, die durch die Modulation der Genexpression in Zellen, Geweben oder im Organismus behandelt werden können.

Gegenstand der Sehrift US20030167030A1 sind Methoden und respiratorische Kompositionen zur Altenulerung der Expression von Targetgenen in vivo unter Verwendung von small-interfering RNAs; insbesondere zur Veränderung des Wachstums, Überlebens oder der Differenzierung von Zellen für therapeutische und kosmetische Anwendungen unter Einsatz pulmonaler oder nasaler Applikationsformen.

Die Schrift DE 10148393 offenbart ein kosmatisches Reinigungsmittol, welches eine Kombination aus Pack-und Applikationsmitteln und einer flüssigen Reinigungszubereitung ist und mit Hilfe des Applikators In einen stabilen Schaum verwandelt wird.

All dieses weist keinen Weg zu Zubereitungen, die sich zur Behandlung und Prophylaxe des verminderten (Haarausfall, Haarminlaturisierung) Wachstums von Haaren der Kopfhaut oder des Körpers eignen, indem der Haarwuchs durch die erfindungsgemäßen Oligoribonukleotide in gewünschter Weise moduliert wird. Ausgehend hiervon stellte sich die Aufgabe, Zubereitungen bereitzustellen, die diese Wirkung aufweisen.

Bekannte Haarbehandlungsmittel haben oft Nachteile. Häufig ist ihre Wirkung nicht zufrieden stellend oder sie sind gesundheitlich nicht unbedenklich, gerade bei ständiger Anwendung.

Wünschenswert sind daher insbesondere topische kosmetische Zubereitungen, die in gewünscht induzierender oder inhibierender Art und Weise auf das Haarwachstum Einfluss nehmen.

Eine Aufgabe der vorliegenden Erfindung war die Bereitstellung von Zusammensetzungen, die eine wirksame Behandlung und Prophylaxe des verminderten (Haarausfall, Haarminiaturisierung) Wachstums von Haaren und insbesondere die Kräftigung der Haare ermöglichen ohne die Nachteile des Standes der Technik zu zeigen.

Eine weitere Aufgabe der Erfindung bestand darin, bessere Mittel zur Beeinflussung des Haarwachstums bereitzustellen.

Es hat sich für den Fachmann nicht vorhersehbar herausgestellt, dass bestimmte Oligoribonukleotide, die den Abbau der mRNA von Strukturen induzieren, die in stimulierender Art und Weise auf den Haarzyklus Ein-fluss nehmen, wobei die Strukturen Proteine und Enzyme darstellen und die Strukturen den Transforming Growth Faktor beta-Superfamlie (TGFbeta)
darstellen,
den Mängeln des Standes der Technik abhelfen.

Diese Aufgabe wird durch Oligoribonukteotide gelöst, die in der Lage sind, die Expression der Gene von Haarzyklus regulierenden Proteinen oder Enzymen zu inhibieren.

Gegenstand der Erfindung ist die Verwendung von Oligoribonukleotiden, die den Abbau der mRNA von Proteinen und Enzymen induzieren, die in stimulierender Art und Weise auf den Haarzyklus Einfluss nehmen zur Herstellung kosmetischer oder dermatologischer Zubereitungen für die Behandlung und Prophylaxe des verminderten (Haarausfall, Haarminiaturisierung) Wachstums von Haaren der Kopfhaut oder des Körpers, wie sie beispielsweise mit altersbedingten Haarwuchsveränderungen einhergehen.

Durch die topische Applikation von doppelsträngigen RNA-Molekülen (RNAI) in kosmetischen Formulierungen kann sowohl die Neubildung von Skalphaaren verbunden mit einer Verlängerung der Wachstumsphase der Haarfollikel erreicht (Haarkräftigung) als auch der umgekehrte Effekt (Haarminiaturisierung) erzielt werden. Dies wird insbesondere ermöglicht durch die selektive Inhibition der Translation des TGFbeta-1, des Androgen-Rezeptors, des Sonic Hedgehog-Gens oder des Gens des Insulin ähnlichen Wachstumsfaktors 1 anhand spezifischer und effizient wirkender siRNA-Moleküle. Dies ermöglicht eine gezielte Einflussnahme auf den Haarzyldus und erlaubt eine gerichtete Prophylaxe bzw. Therapie unerwünschter Haarzustände wie Haarverlust/dünnes Haar

Es wurde weller gefunden, dass es bevorzugt ist, wenn die Strukturen der TGFbeta-Faktoren aus der Gruppe

| | |
|---|---|
| TGFbeta-1 | NM000660 P03958 (EC 3.4_24.7) |
| TGFbete-2 | NM003238 |
| TGFbeta-3 | BT007287 |
| TGFbeta-R I | XM005591 |
| TGFbeta-R II | BC040499 gewählt werden. |

Die TGFbeta-Superfamille enthält eine Gruppe von sekretierten Signalmolekülen, die das Zellwachstum und die Zelldifferenzlerung und eine bedeutende Rolle bei der Regulation des Haarzyklus spielen. TGFbeta Proteine werden Haarzyklus abhängig exprimiert (Seiberg et al, 1995). Die Aktivität dieser Proteine ist für den Transfer des anagenen Haarfollikels in das Catagen/ Telogen verantwortlich (Foitzik et al., 2000). Untersuchungen an Mäusen, die regulierbar TGFbeta1 exprimieren zeigten, dass eine unnalürliche, konstante Induktion der TGFbeta1-Expression Haarausfail verursacht. Diese Alopezie lässt sich auf einen Telogen-Arrest zurückführen (Liu et al., 2001) und wird deutlich durch eine stark erhöhte Apoptose-Rate follikulärer Zellen. Auch bei in vitro kultivierten humanen Haarfollikein konnte ein preliferations-Inhibitorischer TGFbela1-Effekt nachgewiesen werden (Hoffmann et al., 1998). Der genaue Mechanismus, der der prolitefations-inhibitorischen Wirkung auf die Haarwurzeln (Anage-Catagen-Transit) zugrunde liegt, ist noch nicht vollständig geklärt.

Weiter ist es bevorzugt, wenn in solchen Oligoribonukleotiden ihre Zielsequenz oder Zielsequenzen die codierenden Bereiche (cDNA) der jeweiligen Gene darstellt, besonders bevorzugt diejenigen Regionen der codierenden Bereiche, die 60 bis 100 Nukleotide stromabwärts des Startcodons liegen.

Weiter ist es bevorzugl, wenn es sich um doppelsträngige RNA-Moleküle (dsRNAs) handell, die zur Sequenz des Zielgens, bzw. einem Abschnitt davon, homolog sind, d.h. hinsichtlich Sense- und Antisense-Strang mit dem Zielgen übereinstimmen.

Weiter ist es bevorzugt, wenn die dsRNA nicht vollständig mit der Zielsequenz identisch ist, bevorzugt in der Weise, dass bezogen auf eine Länge von 20 Basenpaaren vorzugsweise maximal 0 bis 2, besonders bevorzugt 0 bis 1 und ganz besonders bevorzugt keine Abweichungen von der Zielsequenz auftreten. D.h. es sind maximal 0 bis 2 und insbesondere maximal 0 bis 1 Basenpaare gegen andere Basenpaare ausgetauscht.

Weiter ist es bevorzugt, wenn die Oligoribonukleotide eine Länge von 15 bis 49 Nukleotiden, vorzugsweise 17 bis 30, besonders bevorzugt 19 bis 25 und ganz besonders bevorzugt von 20 bis 23 Nukleotiden aufweisen.

Weiter ist es bevorzugt, wenn es sich um doppelsträngige Oligoribonukleotide handelt, die am 3'·Ende von jedem Strang einen Überhang von 1 bis 6, vorzugsweise 1 oder 2 Nukleotiden aufweisen.

Weiter ist es bevorzugt, wenn die erfindungsgemäßan Oligoribonukleotide zu einem solchen Abschnitt des Zielgens und insbesondere der entsprechenden doppelträngigen cDNA homolog sind, dessen sense-Strang 5'-seitig durch zwei Adenosinreste (A) und 3'-seitig durch zwei Thymidinreste (T), einen Guanosin- (G) und einen Cytosinrest (C) oder einen Thymidin- und einen Cytidinrest (C) begrenzt wird.

Weiter ist es bevorzugt, wenn sie in Expressionsvektoren integriert werden, insbesondere solchen, die eine Expression der Oligoribonukleotide in Säugerzellen bewirken.

Weiter ist es bevorzugt, wenn solche Oligoribonukleotide auf der Ebene der Zuckerreste, der Nukleobasen, der Phosphatgruppen und/oder des dazwischen befindlichen Skeletts chemisch modifiziert sind.

Weiter ist es bevorzugt, wenn eine oder mehrere Phosphatgruppen durch Phosphothioat-, Methylphosphonat-und/oder Phosphoramidatgruppen ausgetauscht sind.

Weiter ist es bevorzugt, wenn erfindungegemässe Oligoribonukleotide durch den Austausch einer oder mehrerer Ribosereste des Oligoribonukleotids durch Morpholinringe (Morpholin-Oligoribonukleotide) oder durch Aminosäuren (Peptid-Oligoribonukleotide) gekennzeichnet sind.

Weiter ist es bevorzugt, wenn deren Ribosereste durch Amitro- wie NH2, Fluor, Alkyl oder O-Alkylreste, wie OCH3, modifiziert sind.

Weiter ist es bevorzugt, wenn sie α-Nukleoside enthalten.

Weiter ist es bevorzugt, wenn in verkapseller Form, beispielsweise verkapselt in Liposomen oder durch die Zugabe von Cyclodextrinen stabilisiert verwendet werden.

Weiter ist es bevorzugt, wenn erfindungsgemäße Oligoribonukleotide und deren Salzen als wirksamer Bestandteil von pharmazeutischen und kosmetischen Zusammensetzungen, insbesondere solchen zur topischen Anwendung verwendet werden.

Weiter ist es bevorzugt, wenn erfindungsgemäße Zubereitungen 1 bis 5 und insbesondere 1 bis 3 verschiedene Oligoribonukleotide enthalten.

Die Erfindung umfasst auch die Verwendung von erfindungsgemäßen in Oligoribonukleotiden in Zubereitungen zur Behandlung und Prophylaxe alters- und umweltbedingter degenerativer und defizitärer Erscheinungen der Haarfoltikel, der Haut und von Hautanhanggebilden, wie Drüsen, insbesondere der in der Beschreibung näher beschriebenen Symptome.

Die Erfindung umfasst weiter auch die Verwendung von erändungsgemäßen Zubereitungen zum Schutz vor Haarausfall, Haarfollikelminiaturisierung, dünner werdendem Haar, Depigmentierungserschelnungen des Haares, dystrophischen Veränderungen der Haarwurzel insbesondere der Haare auf dem Kopf

Neben den genannten Oligoribonukleotiden sind erfindungsgemäß auch physiologisch verträgliche Salze solcher Oligoribonukleotide geeignet. Im folgenden wird der Einfachheit halber der Begriff Oligoribonukleotid sowohl für die Oligoribonukleotide selbst als auch für deren Salze verwendet, wenn nicht anders angegeben. Der Begriff Oligoribonukleotide schließt auch modifizierte Oligoribonukleotide ein.

Bei den angegebenen Zahlen handelt es sich um die Zuganganummern der NCBI-Datenbank (National Center for Biotechnology Information) des National Institutes of Health.

Gegenstand der Erfindung sind jedoch auch längere Nukleotidfragmente, wie z.B. dsRNAs, die in Ihrer Länge den jeweiligen Ziel-mRNAs bzw, cDNAs entsprechen. Diese können z.B. durch löslichen Drosophila Embryo Extrakt in Fragmente einer Länge von 21 bis 23 Nukleotiden übeführt werden (vgl. WO01/75164), Langkettige dsRNA wird zudem intrazellulär zu kurzen Stücken abgebaut. Allerdings ist die direkte Verwendung lankettiger dsRNA im allgemeinen nicht bevorzugt, da diese in Säugerzellen eine unspezifische Inhibition der Translation bewirken kann.

Die erfindungsgemäßen RNA-Duplexe können glatte (blunt ends) oder überstehende (sticky ends) Enden aufweisen. Als besonders wirksam haben sich doppetsträngige Oligoribonukleotide erwiesen, die am 3'-Ende von jedem Strang einen Überhang von 1 bis 6, vorzugsweise 1 oder 2 Nukleotiden aufweisen. Bei den überstehenden Nukleotiden handelt es sich vorzugsweise um 2'-Desoxynukleotide, besonders bevorzugt 2'-Desoxythymidinreste. Durch die Verwendung der 2'-Desoxynukleotide lassen sich die Kosten der RNA-Synthese reduzieren und die Widerstandsfähigkeit der RNA gegenüber dem Nukleaseabbau erhöhen. Die überstehenden Nukleotide müssen nicht zwangsläufig die zur Zielsequenz homologen Nukleotide sein und bleiben daher bei den oben definierten Abweichungen von der Zielsequenz unberücksichtig. Bevorzugt sind allerdings Oligoribonukleotide mit kurzen Überstanden, ins-besondere von 2 Nukleotiden, bei denen die überstehenden Nukleotide des antisense-Strangs der dsRNA zur Zielsequenz komplementär sind.

Als besonders wirksam haben sich Oligoribonukleotide erwiesen, die zu einem solchen Abschnitt des Zielgens und insbesondere der entsprechenden doppelsträngigen cDNA homolog sind, dessen sense-Strang 5'-seitig durch zwei Adenosinreste (A) und 3'-seitig durch zwei Thymidinreste (T), einen Guanosin- (G) und einen Cytosinrest (C) oder einen Thymidin- und einen Cytidinrest (C) begrenzt wird. Der durch AA und TT, AA und GC bzw. AA und TC begrenzte Abschnitt weist vorzugsweise eine Länge von 19 bis 21, insbesondere 19 Nukleotiden auf und hat demnach die allgemeine Form AA(N19-21)TT, AA(N19-21)GC oder AA(N19-21)TC, wobei N für ein Nukleotid steht. Weiter bevorzugt sind Oligoribonukleotide, die zu einem Abschnitt des Zielgens bzw. der entsprechenden doppelsträngigen cDNA komplementär sind, der die allgemeine Form AA(N19) bis AA(N21) hat. Hierbei sind Oligoribonukleotide, die zu dem N19-21-Fragment der genannte Bereiche homolog sind, besonders bevorzugt. Die besonders bevorzugten Oligoribonukleotide weisen somit eine Länge von 19 bis 21 Basenpaaren auf, wobei die diese Oligoribonukleotide bildenden Einzelstränge 3'-seitig vorzugsweise jeweils zwei zusätzliche 2'-Desoxynukleotide, insbesondere zwei 2'-Desoxythymidinreste aufweisen, so dass die dsRNA 19 bis 21 Basenpaare und pro Strang zwei überstehende 2'-Desoxynukleotide umfaßt.

Sollte das Zielgen keinen Bereich der Form AA(N19-21) enthalten, wird nach Bereichen der Form NA(N19-21) oder einem beliebigen Fragment der Form N19-21 gesucht. N19-21-Fragmente, die z.B. von AA und TT begrenzt werden, sind zwar bevorzugt, grundsätzlich sind erfindungsgemäß jedoch alle dsRNA-Fragmente geeignet, die zu der Zielsequenz homolog sind.

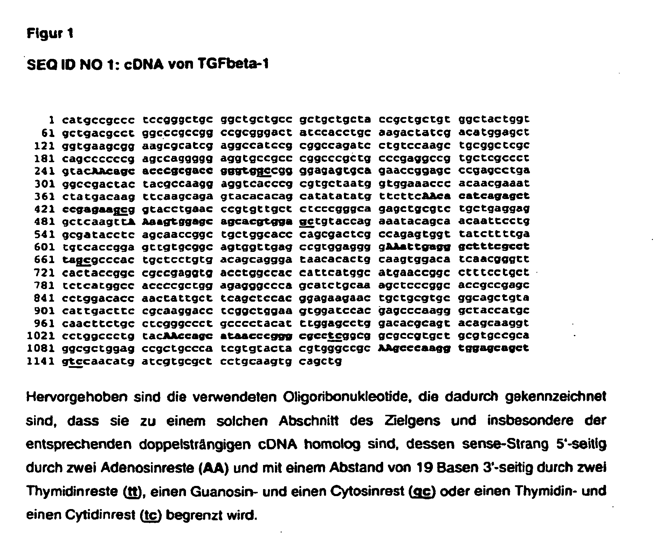

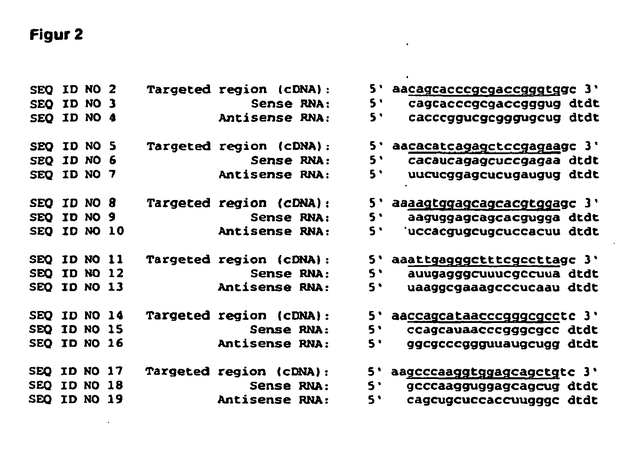

Die Figur 1 zeigt die einzelsträngige cDNA des Wachstumsfaktors TGFbeta-1 (SEQ ID NO 1) in der alle Fragmente der Form AA-N19-TC und AA-N19-GC optisch hervorgehoben sind. In Figur 2 sind diese Fragmente (targeted region) zusammen mit den entsprechenden homologen (senseRNA) und komplementären (antisenseRNA) RNA-Einzelsträngen dargestellt. Gezeigt sind einzelsträngige RNAs, die 3'-seitig durch zwei Desoxythymidinreste (dt) modifiziert sind. Die Hybridisierung von zwei komplementären einzelsträngigen RNAs ergibt dsRNA mit überstehenden 3'-Enden, die durch jeweils zwei 2'-Desoxythymidinreste gebildet werden.

Das Gen des Wachstumsfaktors TGFbeta-1 gehört zu den bevorzugten Zielgenen für die erfindungsgemäßen Oligoribonukleotide.

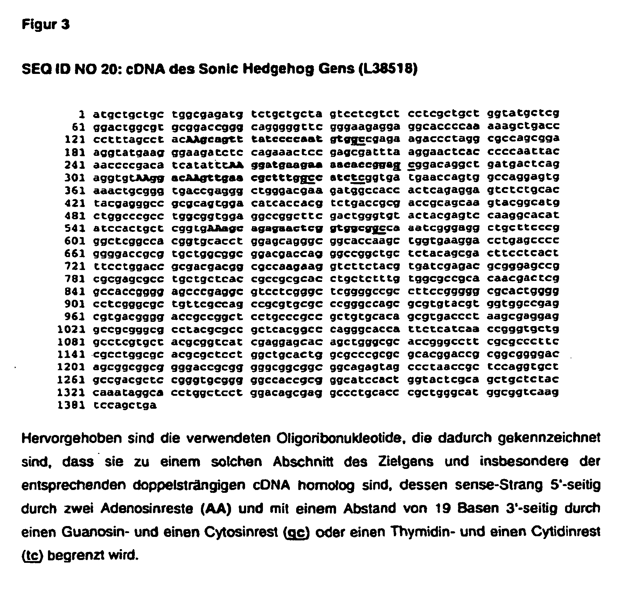

In figur 3 ist die einzelsträngige cDNA des Sonic Hedgehog Gens (SEQ ID NO 20) zu sehen.

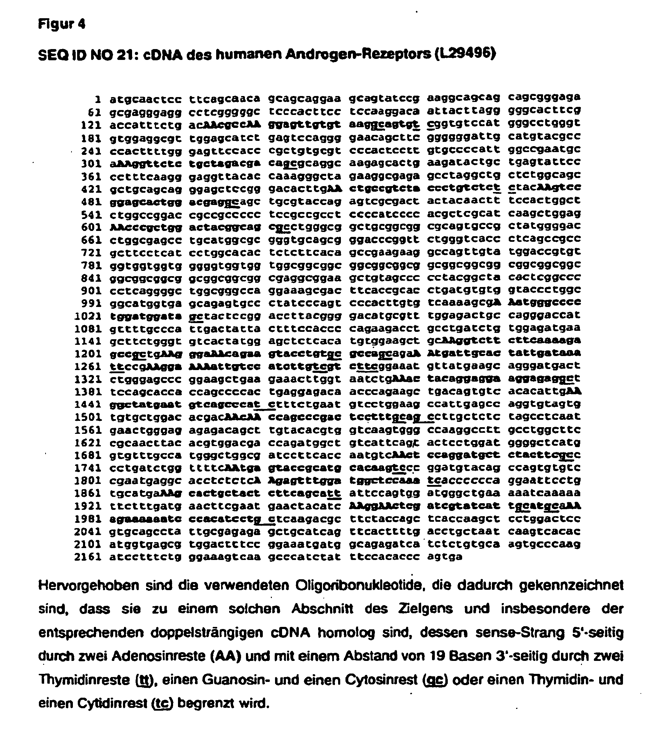

Figur 4 zeigt die einzelsträngige cDNA des Androgen Rezeptors (SEQ ID NO 21)

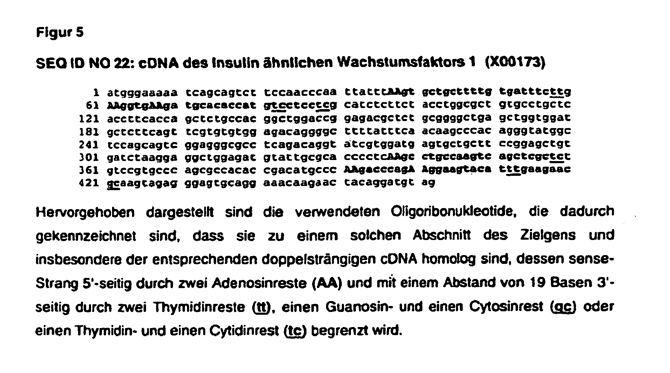

Figur 5 zeigt die einzelsträngige cDNA des Insulin ähnlichen Wachstumsfaktors 1 (SEQ ID NO 22).

Die erfindungsgemäßen Oligoribonukleotide könnten vorteilhaft auch in Expressionsvektoren integriert werden, insbesondere solchen, die eine Expression der Oligoribonukleotide in Säugerzellen bewirken. Auf diese Weise läßt sich selbst bei einem intrazellulären Abbau der Oligoribonukleotide eine stabile Inhibierung der Expression des Zielgens erreichen, da durch die vektorgestützte Synthese ständig Oligoribonukleotide nachgeliefert werden. In einen Vektor können eine oder mehrere Kopien einer dsRNA integriert werden, aber auch jeweils eine oder mehrere Kopien von zwei oder mehr unterschiedlichen dsRNAs. Geeignete Vektorsysteme werden z.B. von Brummelkamp et al., a.a. O. beschrieben. Bevorzugt sind Säuger-Expressionsvektoren, insbesondere solche, die einen Polymerase III H1-RNA-Promotor und 5 bis 9 sogenannte Loops, die aus einer erfindungsgemäßen dsRNA und einer gleichlangen Sequenz, die zur der erfindungsgemäßen dsRNA revers komplementär ist und als Spacer dient, gebildet werden, und ein Terminationssignal von 5 aufeinanderfolgenden Thymidinresten enthalten. Die Vektoren enthalten somit 5 bis 9 Kopien des jeweiligen dsRNA-Moleküls. Hierbei kann es sich dsRNAs handeln, die für 1 Zielgen spezifisch sind, oder um dsRNAs, die für mehrere unterschiedliche Zielgene spezifisch sind.

Die erfindungsgemäßen Oligoribonukleotide können in Form der unmodifizierten Oligoribonukleotide vorliegen. Vorzugsweise handelt es sich jedoch um Oligoribonukleotide, die auf der Ebene der Zuckerreste, der Nukleobasen, der Phosphatgruppen und/oder des dazwischen befindlichen Skeletts chemisch modifiziert sein, um beispielsweise die Stabilität der Oligoribonukleotide in kosmetischen oder dermatologischen Zubereitungen und/oder in der Haut zu erhöhen, z.B. gegenüber einem nukleolytischem Abbau, um die Penetration der Oligoribonukleotide in die Haut und die Zelle zu verbessern, um die Wirksamkeit der Oligoribonukleotide günstig zu beeinflussen und/oder die Affinität zu den zu hybridisierenden Sequenzabschnitten zu verbessern.

Bevorzugt sind Oligoribonukleotide, bei denen eine oder mehrere Phosphatgruppen durch Phosphothioat-, Methylphosphonat- und/oder Phosphoramidatgruppen, wie z.B. N3'→P5'-Phosphoramidatgruppen, ausgetauscht sind. Besonders bevorzugt sind Oligoribonukleotide bei denen Phosphatgruppen durch Phosphothioatgruppen ausgetauscht sind. Es können eine oder mehrere der Phosphatgruppen des Oligoribonukleotids modifiziert sein. Bei einer teilweisen Modifikation werden vorzugsweise endständige Gruppen modifiziert, Oligoribonukleotide bei denen alle Phosphatgruppen modifiziert sind, sind jedoch besonders bevorzugt. Dies gilt sinngemäß auch für die im folgenden beschriebenen Modifikationen.

Bevorzugte Zuckermodifikationen umfassen den Austausch einer oder mehrerer Ribosereste des Oligoribonukleotids durch Morpholinringe (Morpholin-Oligoribonukleotide) oder durch Aminosäuren (Peptid-Oligoribonukleotide). Vorzugsweise sind sämtliche Ribosereste des Oligoribonukleotids gegen Aminosäurereste und insbesondere Morpholinreste ausgetauscht.

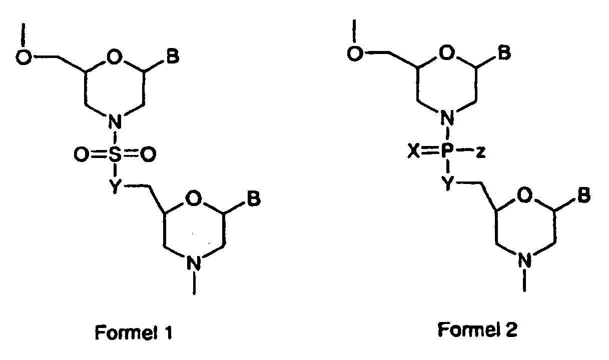

Besonders bevorzugt sind Morpholin-Oligoribonukleotide bei denen die Morpholinreste über Sulfonyl- oder vorzugsweise Phosphorylgruppen miteinander verbunden sind, wie in Formel 1 oder 2 zu sehen ist:
B steht für eine modifizierte oder nicht modifizierte Purin- oder Pyrimidinbase, vorzugsweise für Adenin, Cytosin, Guanin, oder Uracil,
X steht für O oder S, vorzugsweise O,
Y steht für O oder N-CH3, vorzugsweise O,
Z steht für Alkyl, O-Alkyl, S-Alkyl, NH2, NH(Alkyl), NH(O-Alkyl), N(Alkyl)2, N(Alkyl)(O-Alkyl), vorzugsweise N(Alkyl) 2, wobei Alkyl für lineare oder verzweigte Alkylgruppen mit 1 bis 6 vorzugsweise 1 bis 3 und besonders bevorzugt 1 oder 2 Kohlenstoffatomen steht.

Die Formeln 1 und 2 stellen jeweils nur einen Ausschnitt aus einer Oligoribonukleotidkette dar.

Ganz besonders bevorzugt sind Morpholin-Oligoribonukleotide bei denen die Morpholinreste über Phosphorylgruppen miteinander verbunden sind, wie in Formel 2 gezeigt ist, bei denen X für O, Y für O und Z für N(CH3) 2 steht.

Weiterhin können die Ribosereste durch Amino-, wie NH2, Fluor, Alkyl oder O-Alkylreste, wie OCH3, modifiziert werden, wobei 2'-modifizierte Oligoribonukleotide besonders bevorzugt sind. Beispielhafte Modifikationen sind 2'-Fluoro-, 2'-Alkyl-, 2'-O-Alkyl-, 2'-O-Methoxyethyl-Modifikationen, 5'-Palmitat-Derivate und 2'-O-Methylribonukleotide.

Die Modifizierung der Nukleotide von dsRNA wirkt in der Zelle einer Aktivierung der Proteinkinase PKR entgegen, die von doppelsträngiger RNA abhängig ist. Hierdurch wird eine unspezifische Inhibition der Translation vermieden. Zu diesem Zweck eignet sich insbesondere die Substitution mindestens einer 2'-Hydroxylgruppe der Nukleotide der dsRNA durch eine 2'-Amino- oder eine 2'-Methylgruppe. Weiterhin kann mindestens ein Nukleotid in mindestens einem Strang der dsRNA durch ein sogenanntes "locked nucleotide" ersetzt sein, das einen chemisch modifizierten Zuckerring enthält. Eine bevorzugte Modifikation des Zuckerrings ist eine 2'-O, 4'-C-Methylenbrücke. dsRNA, die mehrere "locked nucleotides" enthält, ist bevorzugt.

Wenn nicht anders angegeben, steht Alkyl hierin vorzugsweise für lineare, verzweigte oder cyclische Alkylgruppen mit 1 bis 30, vorzugsweise 1 bis 20, besonders bevorzugt 1 bis 10 und ganz besonders bevorzugt 1 bis 6 Kohlenstoffatomen. Verzweigte und cyclische Reste weisen naturgemäß mindestens 3 Kohlenstoffatome auf, wobei cyclische Reste mit mindestens 5 und insbesondere mindestens 6 Kohlenstoffatomen bevorzugt sind.

Ebenso können Oligoribonukleotide verwendet werden, die α-Nukleoside enthalten.

Geeignete Basenmodifikationen werden z.B. in der US 6,187 578 und der WO 99/53101 beschrieben, auf die hiermit ausdrücklich Bezug genommen wird. Als vorteilhaft hat sich eine Modifikation eines oder mehrerer Pyrimidine in Position 5 mit I, Br, Cl, NH3 und N3 erwiesen.

Die Synthese modifizierter und nicht modifizierter Oligoribonukleotide sowie weitere geeignete Modifikationsmöglichkeiten sind in der Literatur beschrieben. Zudem wird die Herstellung modifizierter und nichtmodifizierter Oligoribonukleotide inzwischen auch von zahlreichen Firmen als Dienstleitung angeboten, beispielsweise von den Firmen Dharmacon, 1376 Miners Drive#101, Lafayette, CO 80026, USA, Xeragon Inc., Genset Oligos und Ambion. Die Herstellung von Oligoribonukleotiden wird darüber hinaus auch in der US 5,986,084 beschrieben.

Zur Erhöhung der Stabilität und/oder der Penetration können die Oligoribonukleotide auch in verkapselter Form verwendet werden, beispielsweise verkapselt in Liposomen. Außerdem können sie durch die Zugabe von Cyclodextrinen stabilisiert werden.

Cyclodextrine werden auch als Cycloamylosen und Cycloglucane bezeichnet. Es handelt sich bei den Cyclodextrinen um zyklische Oligosaccharide bestehend aus α-1,4 verknüpften Glucosebausteinen. In der Regel sind sechs bis acht Glucosebausteine (α-,β, bzw. γ-Cyclodextrin) miteinander verbunden. Cyclodextrine werden bei Einwirkung von Bacillus macerans auf Stärke erhalten. Sie besitzen einen hydrophoben Innenraum und eine hydrophile Außenseite. Erfindungsgemäß sind sowohl die Cyclodextrine selbst, insbesondere α-Cyclodextrin, β-Cyclodextrin und γ-Cyclodextrin, als auch Derivate davon geeignet.

Erfindungsgemäß werden das oder die Cyclodextrine in kosmetischen und dermatologischen Zusammensetzungen vorzugsweise in einer Konzentration von 0.0005 bis 20.0 Gew.-%, insbesondere 0,01 bis 10 Gew.- % und besonders bevorzugt in einer Konzentration von 0.1 bis 5.0 Gew.-% eingesetzt.

Es ist erfindungsgemäß vorteilhaft native, polar- und/oder unpolar- substituierte Cyclodextrine einzusetzen. Hierzu gehören vorzugsweise aber nicht ausschließlich Methyl-, insbesondere random-Methyl-β-Cyclodextrin, Ethylsowie Hydroxypropyl-Cyclodextrine, beispielsweise Hydroxypropyl-β-Cyclodextrin und Hydroxypropyl-β-cyclodextrin.
Die erfindungsgemäß besonders bevorzugten Cyclodextrinspezies sind γ-Cyclodextrin und Hydroxypropyl-β-Cylcodextrin.

Liposomen lassen sich auf an sich bekannte Weise unter Verwendung natürlicher Phospholipide, wie z.B. Phosphatidylcholin aus Eiern, Sojabohnen etc., oder synthetischer Phospholipide herstellen (vgl. G. Betageri (Herausgeber), "Liposome Drug Delivery Systems", Lancaster Techonomic Publishing Company 1993; Gregoriadis (Herausgeber), "Liposome Technology", CRC Press). Bevorzugte Verfahren und Materialien zur Herstellung von Liposomen werden in der WO 99/24018 beschrieben.

Doppelsträngige Oligoribonukleotide können zudem modifiziert werden, um einer Dissoziation in die Einzelstränge entgegenzuwirken, beispielsweise durch eine oder mehrere kovalente, koordinative oder ionische Bindungen. Oligoribonukleotide ohne derartige Modifikationen sind jedoch bevorzugt.

Die Nukleotide in den RNA Molekülen können weiterhin auch "non-standard" Nukleotide, wie z.B. nicht natürlich vorkommende Nukleotide oder Desoxyribonukleotide umfassen.

Erfindungsgemäß sind solche Oligoribonukleotide bevorzugt, die die Expression des jeweiligen Zielgens im Verglich zu unbehandelten Zellen um mindestens 40 %, vorzugsweise um mindestens 60 %, besonders bevorzugt um mindestens 80 % und ganz besonders bevorzugt um mindestens 85 % inhibieren. Falls erforderlich, wird zur Messung der Inhibierung die Expression des Zielgens in den Zellen zunächst auf geeignete Weise induziert. Zur Bestimmung der Wirksamkeit der erfindungsgemäßen Oligoribonukleotide werden vorzugsweise tumorale Zellen der Linie HeLaS3 verwendet. Die Oligoribonukleotide werden in die Zellen eingebracht und anschließend, ggf. nach Induktion der Expression des Zielgens, die Expressionsrate des Zielgens in diesen Zellen gemessen und mit derjenigen verglichen,
die in Zellen gefunden wird, die nicht mit dem jeweiligen Oligoribonukleotid transfiziert wurden. Die genauen Bedingungen zur Messung der Inhibition finden sich in Beispiel 1.

Die erfindungsgemäßen Oligoribonukleotide und deren Salze eignen sich besonders als wirksamer Bestandteil von pharmazeutischen und kosmetischen Zusammensetzungen, insbesondere solchen zur topischen Anwendung.

Es hat sich überraschenderweise herausgestellt, dass die Oligoribonukleotide nach dem Aufbringen der Zusammensetzungen auf die Kopfhaut oder Haut die Expression der Gene inhibieren, die für die Inhibition der Wachstumsphase des Haarfollikels verantwortlich sind, und so den Haarwuchs nebenwirkungsfrei steigern oder vermindern und auf diese Weise eine wirksame Behandlung und Prophylaxe des verminderten (Haarausfall, Haarminiaturisierung) wachstums von Haaren der Kopfhaut oder des Körpers ermöglichen, ohne die Nachteile des Standes der Technik zu zeigen. Es wird angenommen, dass diese Wirkung darauf zurückzuführen ist, dass die erfindungsgemäßen Oligoribonukleotide von den Zellen des Haarfollikels aufgenommen werden und intrazellulär den Abbau der mRNAs der genannten Gene durch RNAi induzieren, wobei Einzelheiten des Mechanismus dieser Reaktionskaskade noch nicht bekannt sind. Die Oligoribonukleotide eignen sich daher besonders zur Initiierung des Abbaus von mRNA von Haarwuchs inhibierenden Proteinen oder Enzymen und zur Inhibierung der Expression der Haarwuchs inhibierenden Proteine oder Enzyme in der Kopfhaut oder Haut und insbesondere in Haarfollikelzellen.

Die erfindungsgemäßen Zusammensetzungen können zusätzlich ein oder mehrere Oligoribonukleotide enthalten, die die Expression der Proteinkinase PKR inhibiert und so einer unspezifischen Inhibition der Translation entgegenwirken.

Die erfindungsgemäßen pharmazeutischen oder kosmetischen Zusammensetzungen enthalten vorzugsweise 0,00001 bis 10 Gew.-%, besonders bevorzugt 0,0003 bis 3 Gew.-% und ganz besonders bevorzugt 0,01 bis 1,0 des oder der erfindungsgemäßen Oligoribonukleotide, bezogen auf das Gesamtgewicht der Zusammensetzung. Bei der Verwendung von Oligorbinonukleotiden, die in Vektoren integriert sind, bezieht sich die obige Mengenangabe auf die Masse der in den Vektor integrierten Oligoribonukleotide, die Masse des Vektors selbst wird nicht berücksichtigt.

Erfindungsgemäß sind solche Zusammensetzungen bevorzugt, die ausschließlich solche Oligoribonukleotide enthalten, die die Expression eines oder mehrerer der oben genannten Gene, d.h. von den Haarzyklus inhibierenden Genen und ggf. der Proteinase PKR, und insbesondere der genannten bevorzugten Gene inhibieren. Die erfindungsgemäßen Zusammensetzungen können ein oder vorzugsweise mehrere Oligoribonukleotide enthalten. Hierbei kann es sich um Oligoribonukleotide handeln, die die Expression mehrerer unterschiedlicher Haarzyklus inhibierender Proteine oder Enzyme inhibieren, es können aber auch Gemische von Oligoribonukleotiden eingesetzt werden, die verschiedene Sequenzbereiche ein und desselben Gens oder derselben mRNA eines Haarzyklus inhibierenden Proteins oder Enzyms zum Ziel haben. Bevorzugt sind Zusammensetzungen, die 1 bis 5 und insbesondere 1 bis 3 verschiedene Oligoribonukleotide enthalten. Mischungen von Oligoribonukleotiden, die neben den genannten Haarzyklus inhibierenden Proteinen oder Enzymen und ggf. der Proteinase PKR unspezifisch die Aktivität einer Vielzahl von anderen Haut- bzw. Haarproteinen inhibieren sind unerwünscht, da praktisch keine Kontrolle von Nebenwirkungen möglich ist. Unter Haut- bzw. Haarproteinen werden solche Proteine verstanden, die in der Haut bzw. im Haarfollikel exprimiert werden. Ganz besonders bevorzugt sind Zusammensetzungen, die ein oder mehrere Oligoribonukleotide enthalten, die die Expression eines oder mehrerer TGFbeta-Faktoren inhibieren.

Die Oligoribonukleotide und Zusammensetzungen eignen sich zur Behandlung und Prophylaxe alters- und umweltbedingter degenerativer und defizitärer Erscheinungen der Haarfollikel, der Haut und von Hautanhanggebilden, wie Drüsen, insbesondere der oben beschriebenen Symptome. Sie eignen sich zur kosmetischen und therapeutischen Behandlung degenerativer Zustände des Haarfollikels, die durch endogene und exogene Faktoren, wie Durchblutungsstörungen, verringerte Energieversorgung der Haarwurzel und insbesondere hormonelle Einflüsse hervorgerufen werden. Die erfindungsgemäßen Zusammensetzungen können Schäden des Haarfollikels vorbeugen und vorhandene Schäden dauerhaft und ohne das Risiko von Nebenwirkungen beheben. Zur Bestimmung der Wirksamkeit der erfindungsgemäßen Oligoribonukleotide kann beispielsweise das in der WO02/053773 beschriebene Verfahren verwendet werden.

Die Oligoribonukleotide und Zusammensetzungen eignen sich zur Prophylaxe und Behandlung von Haarausfall, Haarfollikelminiaturisierung, dünner werdendem Haar, Depigmentierungserscheinungen des Haares, dystrophischen Veränderungen der Haarwurzel

Auf Grund ihrer prophylaktischen Wirkung eignen sich die erfindungsgemäßen Oligoribonukleotide und Zusammensetzungen auch hervorragend zur Haar-, Kopfhaut- und Hautpflege.

Weiter eignen sich die erfindungsgemäßen Zusammensetzungen zur Behandlung der durch verminderte Nährstoff- und/ oder Sauerstoffversorgung, z.B. aufgrund altersbedingter Reduktion der perifollikulären Kapillargefäße und der Kapillarschlinge in der follikulären Papille, hervorgerufenen Defizite des Haarfollikels.

Der erfindungsgemäßen Zusammensetzungen eignen sich zur kosmetischen und therapeutischen, d.h. insbesondere dermatologischen Anwendung.

Unter kosmetischer Hautpflege ist in erster Linie zu verstehen, dass die natürliche Funktion der Haut bzw. Kopfhaut als Barriere gegen Umwelteinflüsse (z. B. Schmutz, Chemikalien, Mikroorganismen) und gegen den Verlust von körpereigenen Stoffen (z. B. Wasser, natürliche Fette, Elektrolyte) gestärkt oder wiederhergestellt wird. Wird diese Funktion gestört, kann es zu verstärkter Resorption toxischer oder allergener Stoffe oder zum Befall von Mikroorganismen und als Folge zu toxischen oder allergischen Hautreaktionen kommen. Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen.

Zur kosmetischen Anwendung enthalten die erfindungsgemäßen Zusammensetzungen daher vorzugsweise solche Komponenten, die für die genannten Zwecke geeignet sind. Solche Substanzen sind dem Fachmann an sich bekannt. Beispielsweise können ein oder mehrere Oligoribonukleotide in übliche kosmetische und dermatologische Zubereitungen eingearbeitet werden, welche in verschiedenen Formen vorliegen können.

Zur Anwendung werden die erfindungsgemäßen Zubereitungen vorzugsweise direkt auf die Kopfhaut oder Körperhaut aufgebracht, und zwar in der für solche Mittel bekannten Weise, beispielsweise zweimal täglich.

Geeignet sind z.B. Lösungen, Gele, Salben, Suspensionen oder Emulsionen wie Cremes oder Lotionen mit einem Gehalt an den erfindungsgemäßen Wirkstoffen.

Gemäß einer besonders bevorzugten Ausführungsform liegen die erfindungsgemäßen Zusammensetzungen zur kosmetischen Anwendung als Haarbehandlungsmittel vor, insbesondere als solche, die im Haar verbleiben oder mit längerer Einwirkzeit verwendet werden. Auf diese Weise gelangen die Wirkstoffe in oder auf die Kopfhaut oder in den Bereich der Haarwurzeln. Haarbehandlungsmittel, die nur kurze Zeit mit der Haut oder den Haaren in Berührung kommen, z.B. Shampoos, können beispielsweise höhere Wirkstoffanteile haben.

Haarbehandlungsmittel sind beispielsweise Haarwaschmittel, Haarpflegemittel wie Haarwässer, Frisierhilfsmittel, Haarspülungen, Haarkuren, Kurpackungen, Haarfestiger wie Schaumfestiger, Haarspray, Haarlack, Haarverformungsmittel und Haarfärbemittel.

Es ist weiter besonders bevorzugt, die beschriebenen Oligoribonukleotide in Zubereitungen einzusetzen, die nicht in erster Linie zur Behandlung des Haares vorgesehen sind. Das können alle denkbaren kosmetischen und/oder dermatologischen Zubereitungen sein

Analoges gilt für Hautpflegezubereitungen, die zur Anwendung im Gesicht oder auf den Beinen oder auf sonstigen Körperteilen, auf denen Haarwuchs als störend angesehen wird, eignen. Hierbei liegen, gemäß einer besonders bevorzugten Ausführungsform, die erfindungsgemäßen Zusammensetzungen zur kosmetischen Anwendung als Emulsion vor, z.B. in Form einer Creme, einer Lotion, einer kosmetischen Milch. Diese enthalten neben den genannten Oligoribonukleotiden weitere Komponenten wie z.B. Fette, Öle, Wachse und/oder andere Fettkörper, sowie Wasser und einen oder mehrere Emulgatoren, wie sie üblicherweise für einen solchen Typ der Formulierung verwendet werden.

Emulsionen enthalten in der Regel eine Lipid- oder Ölphase eine wäßrige Phase und vorzugsweise auch einen oder mehrere Emulgatoren. Besonders bevorzugt sind Zusammensetzungen, die darüber hinaus auch ein oder mehrere Hydrocolloide enthalten.

Die erfindungsgemäßen Zusammensetzungen enthalten vorzugsweise 0,001 bis 35 Gew.-%, besonders bevorzugt 2 bis 15 Gew.-% Emulgator, 0,001 bis 45 Gew.-%, besonders bevorzugt 10 bis 25 Gew.-% Lipid und 10 bis 95 Gew.-%, besonders bevorzugt 60 bis 90 Gew.-% Wasser.

Das Weglassen eines einzelnen Bestandteile beeinträchtigt die einzigartigen Eigenschaften der Gesamtzusammensetzung. Daher sind alle angegebenen Bestandteile der erfindungsgemäßen Zubereitungen zwangsläufig erforderlich, um die Erfindung auszuführen.

Die kosmetischen Zubereitungen gemäß der Erfindung können kosmetische Hilfsstoffe enthalten, wie sie üblicherweise in solchen Zubereitungen verwendet werden, z.B. Konservierungsmittel, Bakterizide, desodorierend wirkende Substanzen, Antitranspirantien, Insektenrepellentien, Vitamine, Mittel zum Verhindern des Schäumens, Farbstoffe. Pigmente mit färbender Wirkung, Verdickungsmittel, weich machende Substanzen, anfeuchtende und/oder feuchthaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate, Antioxidantien und insbesondere UV-Absorber.

Vorteilhaft können Zubereitungen gemäß der Erfindung außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar dienen.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Die Zubereitungen gemäß der Erfindung enthalten vorteilhaft Substanzen, die UV-Strahlung im UV-A- und/ oder UV-B-Bereich absorbieren, wobei die Gesamtmenge der Filtersubstanzen z. B. 0,1 Gew.-% bis 30 Gew.-%, vorzugsweise 0,5 bis 20 Gew.-%, insbesondere 1,0 bis 15,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Weitere vorteilhafte UV-A-Filtersubstanzen sind die Phenylen-1,4-bis-(2-benzimidazyl)-3,3'-5,5'-tetrasulfon-säure 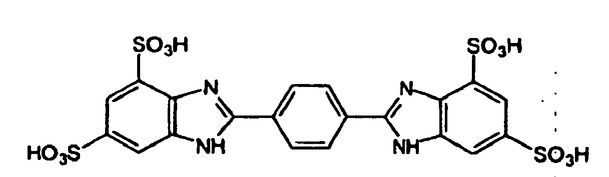 und ihre Salze, besonders die entsprechenden Natrium-, Kalium- oder Trielhanolammonium-Salze, insbesondere das Phenylen-1,4-bis-(2-benzimidazyl)-3,3'-5,5'-tetrasulfonsäure-bis-natriumsalz 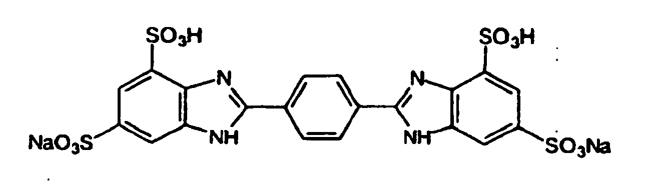 mit der INCI-Bezeichnung Bisimidazylate, welches beispielsweise unter der Handelsbezeichnung Neo Heliopan AP bei Haarmann & Reimer erhältlich ist.

Ferner vorteilhaft sind das 1,4-di(2-oxo-10-Sulfo-3-bomylidenmelhyl)-Benzol und dessen Salze (besonders die entsprechenden 10-Sulfato-verbindungen, insbesondere das entsprechende Natrium-, Kalium- oderTriethanolammonium-Salz), das auch als Benzol-1,4-di(2-oxo-3-bornylidenmethyl-10-sulfonsäure) bezeichnet wird und sich durch die folgende Struktur auszeichnet:

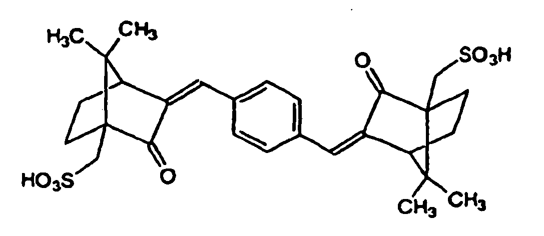

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Bis-Resorcinyltriazinderivate mit der folgenden Struktur: 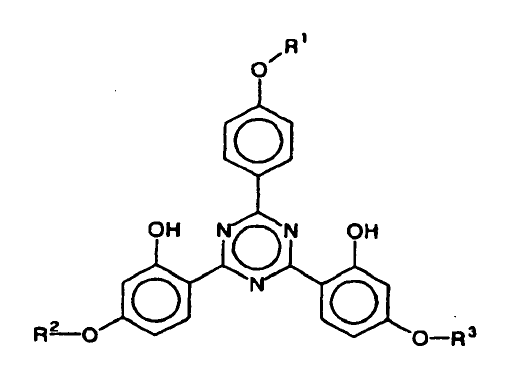 wobei R¹, R² und R³ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist, und das 4,4', 4"-(1,3,5-Triazin-2,4,6-triyltriimino)-tris-benzoesäure-tris(2-ethylhexylester), synonym: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird.

Auch andere UV-Filtersubstanzen, welche das Strukturmotiv 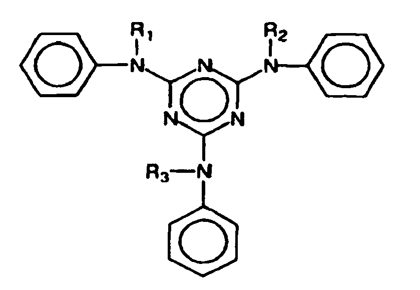 aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel 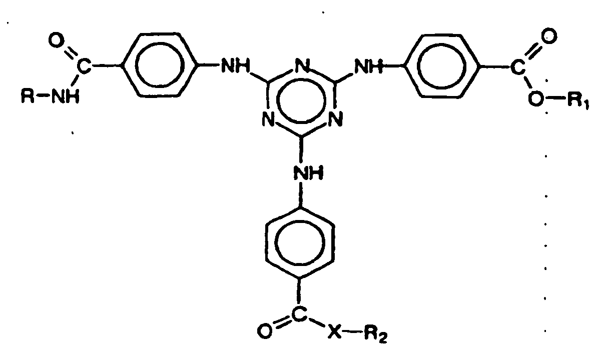 wiedergegeben wird, wobei
- R: einen verzweigten oder unverzweigten C₁-C₁₈-Alkylrest, einen C₅-C₁₂-Cycloalkyl-rest, gegebenenfalls substi- tuiert mit einer oder mehreren C₁-C₄- Alkylgruppen, darstellt,
- X: ein Sauerstoffatom oder eine NH-Gruppe darstellt,
- R₁: einen verzweigten oder unverzweigten C₁-C₁₈-Alkylrest, einen C₅-C₁₂-Cycloalkylrest, gegebenenfalls substitu- iert mit einer oder mehreren C₁-C₄- Alkylgruppen, oder ein Wasserstoffatom, ein Alkalimetallatom, eine Ammo- niumgruppe oder eine Gruppe der Formel 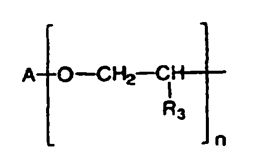 bedeutet, in welcher
A einen verzweigten oder unverzweigten C₁-C₁₈-Alkylrest, einen C₅-C₁₂-Cyclaalkyl- oder Arylrest darstellt, gegebenenfalls substituiert mit einer oder mehreren C₁-C₄- Alkylgruppen,
R₃ ein Wasserstoffatom oder eine Methylgruppe darstellt,
n eine Zahl von 1 bis 10 darstellt,
- R₂: einen verzweigten oder unverzweigten C₁-C₁₈-Alkylrest, einen C₅-C₁₂-Cycloalkylrest, gegebenenfalls substitu- iert mit einer oder mehreren C₁-C₄- Alkylgruppen, darstellt, wenn X die NH-Gruppe darstellt, und einen verzweigten oder unverzweigten C₁-C₁₈-Alkylrest, einen C₅-C₁₂-Cycloalkylrest, gegebenenfalls substitu- iert mit einer oder mehreren C₁-C₄- Alkylgruppen, oder ein Wasserstoffatom, ein Alkalimetallatom, eine Ammo- niumgruppe oder eine Gruppe der Formel 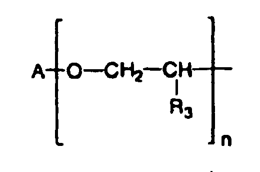
bedeutet, in welcher
- A: einen verzweigten oder unverzweigten C₁-C₁₈-Alkylrest, einen C₅-C₁₂-Cyclo-alkyl- oder Arylrest darstellt, gegebenenfalls substituiert mit einer oder mehreren C₁-C₄- Alkylgruppen,
- R₃: ein Wasserstoffatom oder eine Methylgruppe darstellt,
- n: eine Zahl von 1 bis 10 darstellt,
wenn X ein Sauerstoffatom darstellt.

Eine besonders vorteilhafte UV-Filtersubstanz im Sinne der vorliegenden Erfindung ist ferner ein unsymmetrisch substituiertes s-Triazin, dessen chemische Struktur durch die Formel 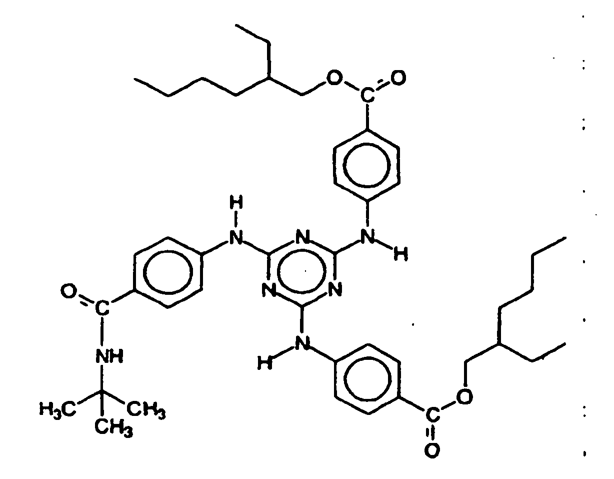 wiedergegeben wird, welches im Folgenden auch als Dioctylbutylamidotriazon (INCI: Dioctylbutamidotriazone) bezeichnet wird und unter der Handelsbezeichnung UVASORB HEB bei Sigma 3V erhältlich ist.

Auch in der Europäischen Offenlegungsschrift EP 775 698 werden vorteilhaft einzusetzende Bis-Resorcinyltriazinderivate beschrieben, deren chemische Struktur durch die generische Formel 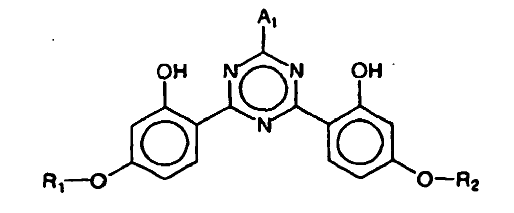 wiedergegeben wird, wobei R₁, R₂ und A₁ verschiedenste organische Reste repräsentieren.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propytoxy)-2-hydroxy-propyloxy)-2-hydroxy]-pheny}-6-(4-methoxypheny)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxy-propyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethyisiloxy-silylpropyloxy)-2-hydroxy]-phenyl}-6-(4-methoxyphenyl)-1,3,5-triazin, das 2,4-Bis-{[4-(2"-methytpropenyloxyl)-2-hydroxy]-phenyl}-6-(4-methoxyphenyl)-1,3,5-triazin und das 2,4-Bis-{[4-(1',1',1',3',5',5',5'-Heptamethylsiloxy-2"-methyl-propyloxy)-2-hydroxy]-phenyl-6-(4-methoxyphenyl)-1,3,5-triazin.

Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol) [INCI: Bisoctyltriazol], welches durch die chemische Strukturformel 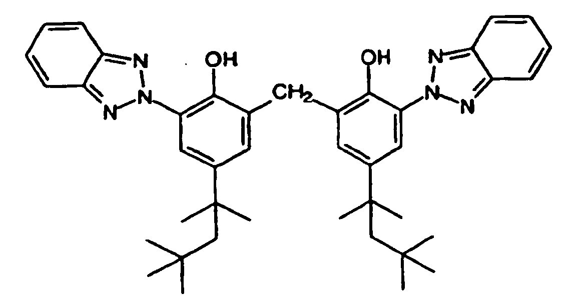 gekennzeichnet ist und unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazo-2-yl)-4-methyl-6-[2-methyl-3-[1,3,3 ,3-tetramethyl-1-[(trimethylsilyl)oxy]disiloxanyl]propyl]-phenol (CAS-Nr.: 155633-54-8) mit der INCI-Bezeichnung Drometrizole Trisiloxane, welches durch die chemische Strukturformel 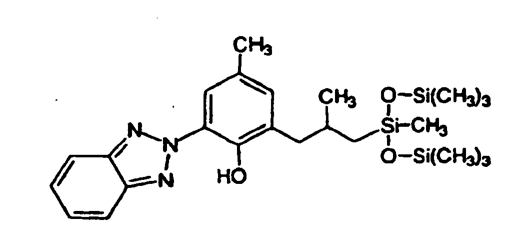 gekennzeichnet ist.

Die UV-B-Filter können öllöslich oder wasserlöslich sein. Vorteilhafte öllösliche UV-B-Filtersubstanzen sind z. B.: 3-Benzylidencampher-Derivate, vorzugsweise 3-(4-Methylbenzyliden)campher, 3-Benzylidencampher; 4-Aminobenzoesäure-Derivate, vorzugsweise 4-(Dimethylamino)-benzoesäure(2-ethylhexyl)ester, 4-(Dimethylamino)ben-zoesäureamylester; 2,4,6-Trianilino-(p-carbo-2''-ethyl-1'-hexyloxy)-1,3,5-triazin; Ester der Benzalmalonsäure, vorzugsweise 4-Methoxybenzalmalonsäuredi(2-ethylhexyl)ester; Ester der Zimtsäure, vorzugsweise 4-Methoxyzimtsäure (2-ethylhexyl)ester, 4-Methoxyzimtsäureisopentylester; Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon sowie an Polymere gebundene UV-Filter.

Vorteilhafte wasserlösliche UV-B-Filtersubstanzen sind z. B. Salze der 2-Phenylbenzimidazol-5-sulfonsäure, wie ihr Natrium-, Kalium- oder ihr Triethanolammonium-Salz, sowie die Sulfonsäure selbst; Sulfonsäure-Derivate des 3-Benzylidencamphers, wie z. B. 4-(2-Oxo-3-bomylidenmethyl)benzolsulfonsäure, 2-Methyl-5-(2-oxo-3-bomyliden-methyl)sulfonsäure und deren Salze.

Eine weiterere erfindungsgemäß vorteilhaft zu verwendende Lichtschutzfiltersubstanz ist das Ethythexyl-2-cyano-3,3-diphenylacrylat (Octocrylen), welches von BASF unter der Bezeichnung Uvinul® N 539 erhältlich ist und sich durch folgende Struktur auszeichnet:

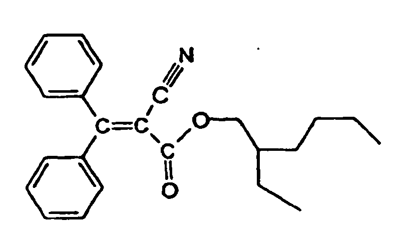

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Ferner kann es gegebenenfalls von Vorteil sein, erfindungsgemäß weitere UV-A-und/oder UV-B-Filter in kosmetische oder dermatologische Zubereitungen einzuarbeiten, beispielsweise bestimmte Salicylsäurederivate wie 4-Isopropylbenzylsalicylat, 2-Ethylhexylsalicylat (= Octylsalicylat), Homomenthylsalicylat.

Die Liste der genannten UV-Filter, die im Sinne der vorliegenden Erfindung eingesetzt werden können, soll selbstverständlich nicht limitierend sein.

Weiterhin können die erfindungsgemäßen Zusammensetzungen Antioxidantien zum Schutz der kosmetischen Zubereitung selbst bzw. zum Schutz der Bestandteile der kosmetischen Zubereitungen vor schädlichen Oxidationsprozessen enthalten.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D, L-Camosin, D-Camosin, L-Camosin und deren Derivate (z.B. Anserin), Carotinoide, Carotine (z.B. α-Carotin, β-Carotin, Lycopin) und deren Derivate, Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl-und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z. B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Alanindiessigsäure, Flavonoide, Polyphenole, Catechine, Vitamin C und Derivate (z.B. Ascorbylpalmitat, Mg-Ascorbylphosphat, Ascorbylacetat), Tocopherole und Derivate (z.B. Vitamin-E-acetat), sowie Koniferylbenzoat des Benzoëharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze. Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Erfindungsgemäß enthalten kosmetische und dermatologische Zubereitungen vorteilhaft außerdem anorganische Pigmente auf Basis von Metalloxiden und/oder anderen in Wasser schwerlöslichen oder unlöslichen Metallverbindungen, insbesondere der Oxide des Titans (TiO₂).Zinks (ZnO), Eisens (z.B. Fe₂O₃). Zirkoniums (ZrO₂), Siliciums (SiO₂), Mangans (z.B. MnO), Aluminiums (Al₂O₃), Cers (z.B. Ce₂C₃), Mischoxiden der entsprechenden Metalle sowie Abmischungen aus solchen Oxiden. Besonders bevorzugt handelt es sich um Pigmente auf der Basis von TiO₂.

Es ist besonders vorteilhaft im Sinne der vorliegenden Erfindung, wenngleich nicht zwingend, wenn die anorganischen Pigmente in hydrophober Form vorliegen, d.h., dass sie oberflächlich Wasser abweisend behandelt sind. Diese Oberflächenbehandlung kann darin bestehen, dass die Pigmente nach an sich bekannten Verfahren mit einer dünnen hydrophoben Schicht versehen werden.

Eines solcher Verfahren besteht beispielsweise darin, dass die hydrophobe Oberflächenschicht nach einer Rektion gemäß

n TiO₂+ m (RO)₃ Si-R' -> n TiO₂ (oberfl.)

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte TiO₂-Pigmente sind beispielsweise unter den Handelsbezeichnungen MT 100 T von der Firma TAYCA, ferner M 160 von der Firma Kemira sowie T 805 von der Firma Degussa erhältlich.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Camosin, natürliche und/oder synthetische Isoflavonoide, Kreatin, Taurin und/oder β-Alanin.

Zubereitungen gemäß der Erfindung können, zumal wenn kristalline oder mikrokristalline Festkörper, beispielsweise anorganische Mikropigmente in die erfindungsgemäßen Zubereitungen eingearbeitet werden sollen, auch anionische, nichtionische und/oder amphotere Tenside enthalten. Tenside sind amphiphile Stoffe, die organische, unpolare Substanzen in Wasser lösen können.

Bei den hydrophilen Anteilen eines Tensidmoleküls handelt es sich meist um polare funktionelle Gruppen, beispielsweise -COO⁻, -OSO₃²⁻, -SO₃⁻, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:

Anionische Tenside weisen als funktionelle Gruppen in der Regel Carboxylat-, Sulfat- oder Sulfonatgruppen auf. In wäßriger Lösung bilden sie im sauren oder neutralen Milieu negativ geladene organische Ionen. Kationische Tenside sind beinahe ausschließlich durch das Vorhandensein einer quaternären Ammoniumgruppe gekennzeichnet. In wäßriger Lösung bilden sie im sauren oder neutralen Milieu positiv geladene organische Ionen. Amphotere Tenside enthalten sowohl anionische als auch kationische Gruppen und verhalten sich demnach in wäßriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

pH=2 RNH2₊CH₂CH₂COOH ×⁻

(X- = beliebiges Anion, z.B. Cl⁻)

pH=7 RNH₂⁺H₂CH₂COO⁻

pH=12 RNHCH₂CH₂COO⁻ B⁺

(B⁺ = beliebiges Kation, z.B. Na⁺)

Typisch für nicht-ionische Tenside sind Polyether-Ketten. Nicht-ionische Tenside bilden in wäßrigem Medium keine Ionen.

Vorteilhaft zu verwendende anionische Tenside sind:

Acylaminosäuren (und deren Salze), wie (1) Acylglutamate, beispielsweise Natriumacylglutamat, Di-TEApalmitoylaspartat und Natrium Caprylicl Capric Glutamat; (2) Acylpeptide, beispielsweise Palmitoyl-hydrolysiertes Milchprotein, Natrium Cocoyl-hydrolysiertes Soja Protein und Natrium-/ Kalium Cocoyl-hydrolysiertes Kollagen; (3) Sarcosinate, beispielsweise Myristoyl Sarcosin, TEA-lauroyl Sarcosinat, Natriumlauroylsarcosinat und Natriumcocoylsarkosinat; (4) Taurate, beispielsweise Natriumlauroyltaurat und Natriummethylcocoyltaurat; (5) Acyllactylate, wie Lauroyllactylat und Caproyllactylat; (6) Alaninate;

Carbonsäuren und Derivate, wie beispielsweise Laurinsäure, Aluminiumstearat, Magnesiumalkanolat und Zinkundecylenat; Ester-Carbonsäuren, beispielsweise Calciumstearoyllactylat, Laureth-6 Citrat und Natrium PEG-4 Lauramidcarboxylat; Ether-Carbonsäuren, beispielsweise Natriumlaureth-13 Carboxylat und Natrium PEG-6 Cocamide Carboxylat;

Carbonsäuren, Ester-Carbonsäuren und Ether-Carbonsäuren enthalten vorzugsweise 1 bis 50 und insbesondere 2 bis 30 Kohlenstoffatome.

Phosphorsäureester und Salze, wie beispielsweise DEA-Oleth-10-Phosphat und Dilaureth-4 Phosphat;

Sulfonsäuren und Salze, wie (1) Acylisethionate, z.B. Natrium-/ Ammoniumcocoylisethionat; (2) Alkylarylsulfonate; (3) Alkylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsulfat; (4) Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat;

Schwefelsäureester, wie (1) Alkylethersulfat, beispielsweise Natrium-, Ammonium-, Magnesium-, MIPA-, TIPA- Laurethsulfat, Natriummyrethsulfat und Natrium C₁₂₋₁₃ Parethsulfat; (2) Alkylsulfate, beispielsweise Natrium-, Ammonium- und TEA- Laurylsulfat.

Vorteilhaft zu verwendende kationische Tenside sind Alkylamine, Alkylimidazole, Ethoxylierte Amine und Quaternäre Tenside sowie Esterquats.

Quaternäre Tenside enthalten mindestens ein N-Atom, das mit 4 Alkyl- oder Arylgruppen kovalent verbunden ist. Dies führt, unabhängig vom pH Wert, zu einer positiven Ladung.

Vorteilhaft sind, Alkylbetain, Alkylamidopropylbetain und Alkyl-amidopropylhydroxysulfain. Die erfindungsgemäß verwendeten kationischen Tenside können ferner bevorzugt gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

Vorteilhaft zu verwendende amphotere Tenside sind (1 )Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat; (2) N-Alkylaminosäuren, beispielsweise Aminopropylalkylglutamid, Alkylaminopropionsäure, Natriumalkylimidodipropionat und Lauroamphocarboxyglycinat.

Vorteilhaft zu verwendende nicht-ionische Tenside sind (1) Alkohole; (2) Alkanolamide, wie Cocamide MEA/ DEN MIPA; (3) Aminoxide, wie Cocoamidopropylaminoxid; (4) Ester, die durch Veresterung von Carbonsäuren mit Ethylenoxid, Glycerin, Sorbitan oder anderen Alkoholen entstehen; (5) Ether, beispielsweise ethoxyliertelpropoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysitoxane, propoxylierte POE-Ether und Alkylpolyglycoside wie Laurylglucosid, Decylglycosid und Cocoglycosid; (6) Sucroseester, -ether; (7) Polyglycerinester, Diglycerinester, Monoglycerinester; (8) Methylglucosester, Ester von Hydroxysäuren.

Vorteilhaft ist ferner die Verwendung einer Kombination von anionischen und/oder amphoteren Tensiden mit einem oder mehreren nicht-ionischen Tensiden.

Die oberflächenaktive Substanz kann in einer Konzentration zwischen 1 und 95 Gew.-% in den erfindungsgemäßen Zubereitungen vorliegen, bezogen auf das Gesamtgewicht der Zubereitungen.

Die Lipidphase der erfindungsgemäßen kosmetischen oder dermatologischen Emulsionen kann vorteilhaft gewählt werden aus folgender Substanzgruppe:
- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen gemäß der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alteinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₂₋₁₅-Alkylbenzoat. 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an zyklischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden. Solche Silicone oder Siliconöle können als Monomere vorliegen, welche in der Regel durch Strukturelemente charakterisiert sind, wie folgt:

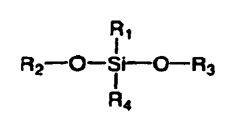

Als erfindungsgemäß vorteilhaft einzusetzenden linearen Silicone mit mehreren Siloxyleinheiten werden im allgemeinen durch Strukturelemente charakterisiert wie folgt: 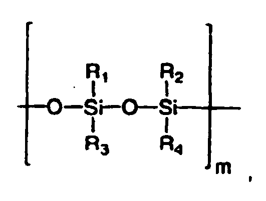 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, dass die Anzahl der unterschiedlichen Reste nicht notwendig auf bis zu 4 beschränkt ist). m kann dabei Werte von 2 - 200.000 annehmen.

Erfindungsgemäß vorteilhaft einzusetzende zyklische Silicone werden im allgemeinen durch Strukturelemente charakterisiert, wie folgt 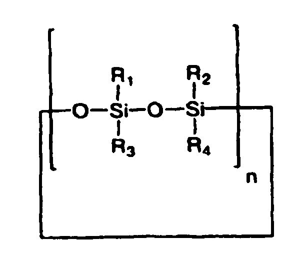 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, dass die Anzahl der unterschiedlichen Reste nicht notwendig auf bis zu 4 beschränkt ist). n kann dabei Werte von 3/2 bis 20 annehmen. Gebrochene Werte für n berücksichtigen, dass ungeradzahlige Anzahlen von Siloxylgruppen im Zyklus vorhanden sein können.

Vorteilhaft wird Cyclomethicon (z.B. Decamethylcyclopentasiloxan) als erfindungsgemäß zu verwendendes Silikonöl eingesetzt. Aber auch andere Silikonöle sind vorteilhaft im Sinne der vorliegenden Erfindung zu verwenden, beispielsweise Undecamethylcyclotrisiloxan, Polydimethylsiloxan, Poly(methylphenylsiloxan), Cetyldimethicon, Behenoxydimethicon.

Vorteilhaft sind ferner Mischungen aus Cyclomethicon und Isotridecylisononanoat, sowie solche aus Cyclomethicon und 2-Ethylhexylisostearat.

Es ist aber auch vorteilhaft, Silikonöle ähnlicher Konstitution wie der vorstehend bezeichneten Verbindungen zu wählen, deren organische Seitenketten derivatisiert, beispielsweise polyethoxyliert und/oder polypropoxyliert sind. Dazu zählen beispielsweise Polysiloxan-polyalkyl-polyether-copolymere wie das Cetyl-Dimethicon-Copolyol, das (Cetyl-Dimethicon-Copolyol (und) Polyglyceryl-4-Isostearat (und) Hexyllaurat)

Besonders vorteilhaft sind ferner Mischungen aus Cyclomethicon und Isotridecylisononanoat, aus Cyclomethicon und 2-Ethylhexylisostearat.

Die wässrige Phase der Zubereitungen gemäß der Erfindung enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate.

Erfindungsgemäß enthalten als Emulsionen vorliegenden Zubereitungen insbesondere vorteilhaft ein oder mehrere Hydrocolloide. Diese Hydrocolloide können vorteilhaft gewählt werden aus der Gruppe der Gummen, Polysaccharide, Cellulosederivate, Schichtsilikate, Polyacrylate und/oder anderen Polymeren.

Erfindungsgemäß enthalten als Hydrogele vorliegenden Zubereitungen ein oder mehrere Hydrocolloide. Diese Hydrocolloide können vorteilhaft aus der vorgenannten Gruppe gewählt werden.

Zu den Gummen zählt man Pflanzen- oder Baumsäfte, die an der Luft erhärten und

Harze bilden oder Extrakte aus Wasserpflanzen. Aus dieser Gruppe können vorteilhaft im Sinne der vortiegenden Erfindung gewählt werden beispielsweise Gummi Arabicum, Johannisbrotmehl, Tragacanth, Karaya, Guar Gummi, Pektin, Gellan Gummi, Carrageen, Agar, Algine, Chondrus, Xanthan Gummi.

Weiterhin vorteilhaft ist die Verwendung von derivatisierten Gummen wie z.B. Hydroxypropyl Guar (Jaguar® HP 8).

Unter den Polysacchariden und -derivaten befinden sich z.B. Hyaluronsäure, Chitin und Chitosan, Chondroitinsulfate, Stärke und Stärkederivate.

Unter den Cellulosederivaten befinden sich z.B. Methylcellulose, Carboxymethylcellulose, Hydroxyethylcellulose, Hydroxypropylmethylcellulose.

Unter den Schichtsilikaten befinden sich natürlich vorkommende und synthetische Tonerden wie z.B. Montmorillonit, Bentonit, Hektorit, Laponit, Magnesiumaluminiumsilikate wie Veegum®. Diese können als solche oder in modifizierter Form verwendet werden wie z.B. Stearylalkonium Hektorite.

Weiterhin können vorteilhaft auch Kieselsäuregele verwendet werden.

Unter den Polyacrylaten befinden sich z.B. Carbopol Typen der Firma Goodrich (Carbopol 980, 981, 1382, 5984, 2984, EDT 2001 oder Pemulen TR2).

Unter den Polymeren befinden sich z.B. Polyacrylamide (Seppigel 305), Polyvinylalkohole, PVP, PVP / VA Copolymere, Polyglycole.

Gemäß einer weiteren bevorzugten Ausführungsform werden die erfindungsgemäß verwendeten Oligoribonukleotide in wäßrige Systeme bzw. Tensidzubereitungen zur Reinigung der Haut und der Haare eingefügt.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Homschicht (auch trans-epidermal water loss (TEWL) genannt) zu reduzieren und/oder die Hydratation der Hornschicht positiv zu beeinflussen. Vorteilhafte Moisturizer im Sinne der vorliegenden Erfindung sind beispielsweise Glycerin, Milchsäure, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure und/oder ein fucosereiches Polysaccharid, welches in den Chemical Abstracts unter der Registratumummer 178463-23-5 abgelegt und z. B. unter der Bezeichnung Fucogel□ 1000 von der Gesellschaft SOLABIA S.A. erhältlich ist.

Glycerin wird bei der Verwendung als Moisturizer vorzugsweise in einer Menge von 0,05-30 Gew.%, besonders bevorzugt sind 1-10%, eingesetzt.

Erfindungsgemäß enthalten als Emulsionen vorliegenden Zubereitungen einen oder mehrere Emulgatoren. Diese Emulgatoren können vorteilhaft gewählt werden aus der Gruppe der nichtionischen, anionischen, kationischen oder amphoteren Emulgatoren.

Unter den nichtionischen Emulgatoren befinden sich
a) Partialfettsäureester und Fettsäureester mehrwertiger Alkohole und deren ethoxylierte Derivate (z. B. Glycerylmonostearate, Sorbitanstearate, Glycerylstearylcitrate, Sucrosestearate)
b) ethoxylierte Fettalkohole und Fettsäuren
c) ethoxylierte Fettamine, Fettsäureamide, Fettsäurealkanolamide
d) Alkylphenolpolyglycolether (z.B. Triton X)

Unter den anionischen Emulgatoren befinden sich
a) Seifen (z. B. Natriumstearat)
b) Fettalkoholsulfate
c) Mono-, Di- und Trialkylphosphosäureester und deren Ethoxylate

Unter den kationischen Emulgatoren befinden sich
a) quaternäre Ammoniumverbindungen mit einem langkettigen aliphatischen Rest z.B. Distearyldimonium Chloride

Unter den amphoteren Emulgatoren befinden sich
a) Alkylamininoalkancarbonsäuren
b) Betaine, Sulfobetaine
c) Imidazolinderivate

Weiterhin gibt es natürlich vorkommende Emulgatoren, zu denen Bienenwachs, Wollwachs, Lecithin und Sterole gehören.

O/W-Emulgatoren können beispielsweise vorteilhaft gewählt werden aus der Gruppe der polyethoxylierten bzw. polypropoxylierten bzw. polyethoxylierten und polypropoxylierten Produkte, z.B.:
- der Fettalkoholethoxylate
- der ethoxylierten Wollwachsalkohole,
- der Polyethylenglycolether der allgemeinen Formel R-O-(-CH₂-CH₂-O-)ₙ-R'.
- der Fettsäureethoxylate der allgemeinen Formel

   R-COO-(-CH₂-CH₂-O-)ₙ-H.
- der veretherten Fettsäureethoxylate der allgemeinen Formel

   R-COO-(-CH₂-CH₂-O-)ₙ-R',
- der veresterten Fettsäureethoxylate der allgemeinen Formel

   R-COO-(-CH₂-CH₂-O-)ₙ-C(O)-R',
- der Polyethylenglycolglycerinfettsäureester
- der ethoxylierten Sorbitanester
- der Cholesterinethoxylate
- der ethoxylierten Triglyceride
- der Alkylethercarbonsäuren der allgemeinen Formel R-O-(-CH₂-CH₂-O-)ₙ-CH₂-COOH nd n eine Zahl von 5 bis 30 darstellen,
- der Polyoxyethylensorbitolfettsäureester,
- der Alkylethersulfate der allgemeinen Formel R-O-(-CH₂-CH₂-O-)ₙ-SO₃-H
- der Fettalkoholpropoxylate der allgemeinen Formel

   R-O-(-CH₂-CH(CH₃)-O-)ₙ-H,
- der Polypropylenglycolether der allgemeinen Formel

   R-O-(-CH₂-CH(CH₃)-O-)ₙR',
- der propoxylierten Wollwachsalkohole,
- der veretherten Fettsäurepropoxylate

   R-COO-(-CH₂-CH(CH₃)-O-)ₙ-R',
- der veresterten Fettsäurepropoxylate der allgemeinen Formel

   R-COO-(-CH₂-CH(CH₃)-O-)ₙ-C(O)-R',
- der Fettsäurepropoxylate der allgemeinen Formel

   R-COO-(-CH₂-CH(CH₃)-O-)ₙ-H,
- der Polypropylenglycolglycerinfettsäureester
- der propoxylierten Sorbitanester
- der Cholesterinpropoxylate
- der propoxylierten Triglyceride
- der Alkylethercarbonsäuren der allgemeinen Formel

   R-O-(-CH₂-CH(CH₃)-O-)ₙ-CH₂-COOH
- der Alkylethersulfate bzw. die diesen Sulfaten zugrunde liegenden Säuren der allgemeinen Formel R-O-(-CH₂-CH
   (CH₃)-O-)ₙ-SO₃-H
- der Fettalkoholethoxylate/propoxylate der allgemeinen Formel

   R-O-Xₙ-Yₘ-H,
- der Polypropylenglycolether der allgemeinen Formel

   R-0-Xₙ-Y_{M}-R',
- der veretherten Fettsäurepropoxylate der allgemeinen Formel

   R-COO-Xₙ₋Yₘ-R',
- der Fettsäureethoxylate/propoxylate der allgemeinen Formel

   R-CCO-Xₙ-Yₘ-H,.

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe derSubstanzen mit HLB-Werten von 11 - 18, ganz besonders vorteilhaft mit mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:

Polyethylenglycol(13)stearylether (Steareth-13), Polyethylenglycol(14)stearylether (Steareth-14), Polyethylenglycol(15)stearylether (Steareth-15). Polyethylenglycol(16)stea-rylether (Steareth-16). Polyethylenglycol(17) stearylether (Steareth-17), Polyethylenglycol-(18)stearylether (Steareth-18), Polyethylenglycol(19)stearylether (Steareth-19), Polyethylenglycol(20)stearylether (Steareth-20),

Polyethylenglycol(12)isostearylether (Isosteareth-12). Polyethylenglycol(13)isostearylether (Isosteareth-13). Polyethylenglycol(14)isostearylether (Isosteareth-14), Polyethylenglycol(15)isostearylether (Isosteareth-15), Polyethylenglycol(16)isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol (20)isostearylether (Isosteareth-20),

Polyethylenglycol(13)cetylether (Ceteth-13), Polyethylenglycol(14)cetylether (Ceteth-14), Polyethylenglycol (15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20).

Polyethylenglycol(13)isocetylether (Isoceteth-13). Polyethylenglycol(14)isocetylether (Isoceteth-14), Polyethylenglycol(15)isocetylether(Isoceteth-15), Polyethylenglycol(16)-isocetylether (Isoceteth-16), Polyethylenglycol(17) isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20).

Polyethylenglycol(12)oleylether (Oteth-12), Polyethylenglycol(13)oleylether (Oleth-13), Polyethylenglycol (14)oleylether (Oleth-14), Polyethylenglycol(15)oleylether (Oleth-15),

Polyethylenglycol(12)laurylether (Laureth-12), Polyethylenglycol(12)isolaurylether (Isolaureth-12).

Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylengtycol(17)cetylstearylether (Ceteareth-17), Polyethylenglycol(18)cetylstearylether (Ceteareth-18), Polyethylenglycol-(19)cetylstearylether (Ceteareth-19), Polyethylenglycol(20)cetylstearylether (Ceteareth-20),

Es ist ferner von Vorteil, die Fettsäureethoxylate aus folgender Gruppe zu wählen:

Polyethylenglycol(20)stearat, Polyethylenglycol(21)stearat, Polyethylenglycol(22)stearat, Polyethylenglycol (23)stearat, Polyethylenglycol(24)stearat, Polyethylenglycol(25)stearat,

Polyethylenglycol(12)isostearat, Polyethylenglycol(13)isostearat, Polyethylenglycol(14)-isostearat, Polyethylenglycol(15)isostearat, Polyethylenglycol(16)isostearat, Polyethylenglycol(17)isostearat, Polyethylenglycol(18) isostearat, Polyethylenglycol(19)isostearat, Polyethylenglycol(20)isostearat, Polyethylenglycol(21)isostearat, Polyethylenglycol(22)isostearat, Polyethylenglycol(23)isostearat, Polyethylenglycol(24)isostearat, Polyethylenglycol(25) isostearat,

Polyethylenglycol(12)oleat, Polyethylenglycol(13)oleat, Polyethylenglycol(14)oleat, Polyethylenglycol(15) oleat, Polyethylenglycol(16)oleat, Polyethylenglycol(17)oleat, Polyethylenglycol(18)oleat, Polyethylenglycol(19)oleat, Polyethylenglycol(20)oleat

Als ethoxylierte Alkylethercarbonsäure bzw. deren Salz kann vorteilhaft das Natriumlaureth-11-carboxylat verwendet werden.

Als Alkylethersulfat kann Natrium Laureth 1-4 sulfat vorteilhaft verwendet werden.

Als ethoxyliertes Cholesterinderivat kann vorteilhaft Polyethylenglycol(30)Cholesterylether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20) glyceryllaurat, Polyethylenglycol(21)glyceryllaurat, Polyethylenglycol-(22)glyceryllaurat, Polyethylenglycol(23)glyceryllaurat, Polyethylenglycol(6)glycerylcaprat/caprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitan-monopalmitat, Polyethylenglycol(20)-sorbitanmonooleat zu wählen.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 - 18 C-Atomen, Diglycerinester gesättigter und/oder ungesättigter, verzweigter und/oder unverzweigter Alkancarbonsäuren einer Kettenlänge von 8 bis 24, insbesondere 12 - 18 C-Atomen, Monoglycerinether gesättigter und/oder ungesättigter, verzweigter und/oder unverzweigter Alkohole einer Kettenlänge von 8 bis 24, insbesondere 12 - 18 C-Atomen, Diglycerinether gesättigter und/oder ungesättigter, verzweigter und/ oder unverzweigter Alkohole einer Kettenlänge von 8 bis 24, insbesondere 12 - 18 C-Atomen, Propylenglycolester gesättigter und/oder ungesättigter, verzweigter und/oder unverzweigter Alkancarbonsäuren einer Kettenlänge von 8 bis 24, insbesondere 12 - 18 C-Atomen sowie Sorbitanester gesättigter und/oder ungesättigter, verzweigter und/oder unverzweigter Alkancarbonsäuren einer Kettenlänge von 8 bis 24, insbesondere 12 - 18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Als übliche kosmetische Trägerstoffe zur Herstellung der desodorierenden Zubereitungen gemäß der erfindungsgemäßen Verwendung können neben Wasser, Ethanol und Isopropanol, Glycerin und Propylenglykol hautpflegende Fett- oder fettähnliche Stoffe, wie Ölsäuredecylester, Cetylalkohol, Cetylstearylalkohol und 2-Octyldodecanol, in den für solche Präparate üblichen Mengenverhältnissen eingesetzt werden sowie schleimbildende Stoffe und Verdickungsmittel, z.B. Hydroxyethyl- oder Hydroxypropylcellulose, Polyacrylsäure, Polyvinylpyrrolidon, daneben aber auch in kleinen Mengen cyclische Silikonöle (Polydimethylsiloxane) sowie flüssige Polymethylphenylsiloxane niedriger Viskosiät.

Es ist von Vorteil, wenn die erfindungsgemäßen Zubereitungen neben der Wirkstoffkombination zusätzlich Adstringetntien bzw. Antitranspirantien, Silikonöle und/oder Puderstoffen enthalten.

Antitranspirantien kommen z. B. Aluminiumchlorhydate in Frage. Hierbei handelt es sich um farblose, hygroskopische Kristalle, die an der Luft leicht zerfließen und beim Eindampfen wäßriger Aluminiumchloridlösungen anfallen. Aluminiumchlorhydrat wird zur Herstellung von schweißhemmenden und desodorierenden Zubereitungen eingesetzt und wirkt wahrscheinlich über den partiellen Verschluß der Schweißdrüsen durch Eiweiß- und/oder Polysaccharidfällung. Neben den Chlorhydraten können auch Aluminiumhydroxylactate sowie saure Aluminium/Zirkoniumsalze eingesetzt werden.
- Aluminium-Salze (der empirischen Summenformel [Al₂(OH)ₘClₙ], wobei m+n=6):
- Aluminium-Salze wie Aluminiumchlorid AlCl₃, Aluminiumsulfat Al₂(SO₄)₃
- Aluminiumchlorhydrat [Al₂(OH)₅Cl] × H₂O
- Standard Al-Komplexe: Locron L (Clariant), Chlorhydrol (Reheis), ACH-303 (Summit), Aloxicoll L (Giulini).
- Aktivierte Al-Komplexe: Reach 501 (Reheis), AACH-324 (Summit)
- Aluminiumsesquichlorhydrat [Al₂(OH)_{4,5}Cl_{1,5} ] × H₂O
- Standard Al-Komplexe: Aluminum Sesquichlorohydrate (Reheis), ACH-308 (Summit), Aloxicoll 31 L (Giulini)
- Aktivierte Al-Komplexe: Reach 301 (Reheis)
- Aluminiumdichlorhydrat [Al₂(OH)₄Cl₂] × H₂O
- Aluminium-Zirkonium-Salze:
- Aluminium/Zirkonium Trichlorhydrex Glycin [Al₄Zr(OH)₁₃Cl₃] × H₂O × Gly
- Standard Al/Zr-Komplexe: Rezal 33GP (Reheis), AZG-7164 (Summit), Zirkonal P3G (Giulini)
- Aktivierte AU/Zr-Komplexe: Reach AZZ 902 (Reheis), AAZG-7160 (Summit), Zirkonal AP3G (Giulini)
- Aluminium/Zirkonium Tetrachlorhydrex Glycin [Al₄Zr(OH)₁₂Cl₄] × H₂O × Gly
- Standard Al/Zr-Komplexe: Rezal 36G (Reheis), AZG-368 (Summit), Zirkonal L435G (Giulini)
- Aktivierte Al/Zr-Komplexe: Reach AZP 855 (Reheis), AAZG-6313-15 (Summit), Zirkonal AP4G (Giulini)
- Aluminium/Zirkonium Pentachlorhydrex Glycin [Al₈Zr(OH)₂₃Cl₅] × H₂O × Gly
- Standard Al/Zr-Komplexe: Rezal 67 (Reheis), Zirkonal L540 (Giulini)
- Aktivierte Al/Zr-Komplexe: Reach AZN 885 (Reheis)
- Aluminium/Zirkonium Octachlorhydrex Glycin [Al₈Zr(OH)₂₀Cl₈] × H₂O × Gly

Ebenso von Vorteil können aber auch Glycin-freie Aluminium/Zirkonium-Salze sein.

Dabei soll die Verwendung der Antitranspirant-Wirker aus den Rohstoffklassen Aluminium- und Aluminium/ Zirkonium-Salzen nicht auf die handelsüblichen zumeist wäßrigen Lösungen, wie z.B. Locron L (Clariant), beschränkt sein, sondern es kann auch von Vorteil sein, die ebenfalls handelsüblichen wasserfreien Pulver derselbigen Rohstoffe durch Einbringung in die beanspruchten Formulierungen zum Einsatz zu bringen, wie z.B. Locron P (Clariant).

Vorteilhaft könnte auch die Verwendung von sog. AT-Salz Suspensionen sein, bei denen pulverförmig vorliegende Aluminium- und Aluminium/Zirkonium-Salze in diversen Ölen dispergiert angeboten werden.

Desweiteren kann es aber auch von Vorteil sein, spezielle Aluminium- und Aluminium/Zirkonium-Salze zum Einsatz zu bringen, die zur Löslichkeitsverbesserung als Glykol-Komplexe angeboten werden.

Weitere vorteilhafte Antitranspirant-Wirker basieren anstelle von Aluminium bzw. Zirkonium auf anderen Metallen, wie z.B. Beryllium, Titan, Hafnium.

Dabei soll die Liste der verwendbaren Antitranspirant-Wirker aber nicht auf metallhaltige Rohstoffe begrenzt sein, sondern von Vorteil sind auch Verbindungen, die Nichtmetalle wie Bor enthalten sowie solche, die dem Bereich der organischen Chemie zuzurechnen sind, wie z.B. Anticholinergika.

Vorteilhaft sind in diesem Sinne auch Polymere, die sowohl metallhaltig als auch metallfrei sein können.

Als Treibmittel für erfindungsgemäße, aus Aerosolbehältern versprühbare dermatologische Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vortiegende Erfindung geeignet wären, auf die aber dennoch wegen bedenklicher Wirkung auf die Umwelt oder sonstiger Begleitumstände verzichtet werden sollte, insbesondere Fluorchlorkohlenwasserstoffe (FCKW).

Zusätzlich enthalten sprühbare Zubereitungen bestimmte Olkomponenten und Alkohole Ethanol und Isopropanol, Glycerin und Propylenglykol hautpflegende Fett- oder fettähnliche Stoffe, wie Ölsäuredecylester, Cetylalkohol, Cetylstearylalkohol und 2-Octyldodecanol, in den für solche Präparate üblichen Mengenverhältnissen eingesetzt werden sowie schleimbildende Stoffe und Verdickungsmittel, z.B. Hydroxyethyl- oder Hydroxypropylcellulose, Polyacrylsäure, Polyvinylpyrrolidon, daneben aber auch in kleinen Mengen cyclische Silikonöle (Polydimethylsiloxane) sowie flüssige Polymethylphenylsiloxane niedriger Viskosiät.

Es ist bei all diesem im Einzelfalle möglich, dass die vorgenannten Konzentrationsangaben leicht über- oder unterschritten werden und dennoch erfindungsgemäße Zubereitungen erhalten werden. Dies kommt angesichts der breit streuenden Vielfalt an geeigneten Komponenten derartiger Zubereitungen für den Fachmann nicht unerwartet, so dass er weiß, dass bei solchen Über- oder Unterschreitungen der Boden der vorliegenden Erfindung nicht verlassen wird.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele

### Beispiel 1: Messung der Inhibition der TGFbeta-1-Expression In humanen dermalen Fibroblasten durch dsRNA

Zur Ermittlung der Wirksamkeit erfindungsgemäßer Oligoribonukleotide wurden humane dermale Fibroblasten (HDF-Zellen) verwendet. Die Expression des Wachstumsfaktors TGFbeta-1 erfolgt von den Zellen endogen.

Hierzu wurden die Zellen (in DMEM mit 10 % foetalem Kälber Serum) am Tag vor der Induktion in einer Dichte von 7x105 Zellen pro 10cm-Schale ausgesät und für 24 Std. im Vollmedium (DMEM + 10% FCS + 1% Glutamax + 1% Pen/Strep) kultiviert. Die Transfektion wurde unter Verwendung eines Gemischs aus kationischen Lipiden (Oligofektamin Reagenz, Invitrogen) durchgeführt. Für den Transfektionsansatz wurden 90 µl der verschiedenen anti-TGFbeta-1 dsRNAs (dsRNAs, die durch Hybridisieren der Sequenzen SEQ ID NOs 15 und 16, der SEQ ID NOs 18 und 19 bzw. der SEQ ID NOs 6 und 7 erhalten wurden, die dsRNAs weisen 3'-ständig jeweils zwei überstehende dT-Reste auf, s. Figur 2) in 1,5 ml Medium gelöst. In einem zweiten Ansatz wurde 36 µl Oligofektamin in 240 µl Medium gelöst und für 5-10 min inkubiert. Nach dieser Inkubationszeit wurden die beiden Ansätze zusammengegeben und für weitere 15-20 min inkubiert. In der Zwischenzeit wurde das Vollmedium von den Zellen entfernt und durch 7 ml Medium ohne Zugabe von Antibiotika oder Serum ersetzt. Der Transfektionsansatz wurde nun auf die Zellen gegeben. Anschließend wurde dieser Transfektionsansatz für 24 Std. auf den primären Fibroblasten belassen. Als Vergleich dienten Proben, die nicht mit dsRNA oder solche, die mit einer nicht das TGFbeta-1 bindenden dsRNA (anti-LaminA/C-Kontroll-Oligoribonukleotide der Firma MWG) transfiziert wurden.

Nach der mikroskopischen Untersuchung der Zellen wurde die Gesamt RNA isoliert. Dazu wurde der Mediumüberstand entfernt und die Zellen einmal mit PBS gespült. Die Zellen wurden mit 200gm PBS und 400µl Lyse-Puffer überdeckt, abgeschabt und in Eppendorf Gefäße überführt. Die RNA-Isolation erfolgte anhand des High Pure RNA Isolation Kits (Roche Diagnostics Corp.) nach den Angaben des Herstellers. Die hierbei wurde an eine Säule gebundene isolierte RNA wurde mit 50 - 100µl Wasser eluiert.

Aus der isolierten RNA wurde der Gehalt an TGFbeta-1 mRNA mit Hilfe der quantitativen TaqMan-Analyse (TaqMan Assays-on-Demand™ Applied Biosystems) ermittelt.

Hierzu wurde in einem 2-Schritt-Verfahren erst die Gesamt-RNA in cDNA umgeschrieben. Die Reverse Transkription wurde mit Hilfe des *High Capacity cDNA Archive* Kits (Applied Biosystems) nach Angaben des Herstellers durchgeführt.

Bei der quantitativen PCR wurden 1 µl der cDNA-Reaktion (entspricht 10 ng Gesamt-RNA) mit 10,25 µl Wasser, 12,5 µl TaqMan Universal Master Mix (mit AmptiTaq-Gotd-Potymerase) und 1,25 µl Target Mix (spezifische Primer und Fluoreszenz-markierte Probe) für 18S-RNA als Kontrolle bzw. TGF-β1 gemischt. Im ABI PRISM 7700 wurden die Proben erst 2 min bei 50°C zur Aktivierung der Uracil-N-Glycolase inkubiert, woran sich eine 10minütige Inaktivierung bei 95°C anschloss. Die PCR erfolgte über 40 Zyklen bei 95°C (15 s) (Denaturierung) und 60 °C (1 min) (Annealing).
Die Fluoreszenzänderung wird vom ABI PRISM 7700 während der PCR detektiert.

Die Quantifizierung der Genexpression erfolgte über den C₁-Wert (threshold cycle). Die Relativierung erfolgte über die endogene Kontrolle der 18s RNA.

Aus den Dreifachbestimmungen von Ziel-Gen (TGFbeta-1) und endogener Kontrolle (18sRNA) pro Probe wurden die Mittelwerte gebildet und diese dann dividiert (ng Probe/ng Kontrolle). Auf diese Weise wurden normalisierte, einheitslose Werte der relativen quantitativen Expression des Ziel-Gens erhalten.

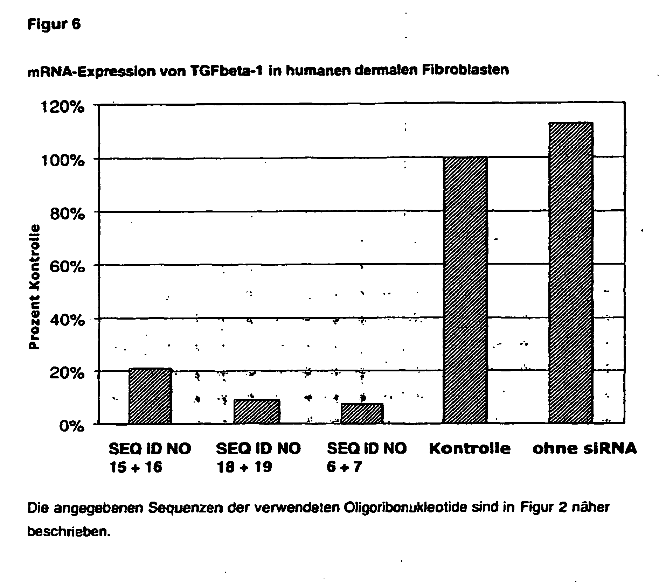

Die Ergebnisse sind in Figur 6 graphisch dargestellt. Hier ist die über den 18S rRNA-Gehalt normierte relative mRNA-Konzentration des Wachstumsfaktors TGFbeta-1 im prozentualen Verhältnis zu Kontrolle zu sehen. Die Transfektion der Zellen mit der Transfektionsreagenz allein ("ohne siRNA") sowie mit den Oligoribonukleotiden gegen Lamin A/C ("Kontrolle") bewirkt keine Änderung des TGFbeta-1 mRNA-Gehaltes. Die Transfektion mit den drei unterschiedlichen Oligoribonukleotidpaaren gegen TGFbeta-1 führt hingegen jeweils zu einer Abnahme der TGFbeta-1-Konzentration ("SEQ ID NOs "15+16", "18+19" bzw. "6+7"). Die Inhibition beträgt ca. 80-90 % im Vergleich zu der Kontrolle und zu den nicht mit siRNA transfizierten Zellen.

In Figur 1 ist die codierende Sequenz von TGFbeta-1 dargestellt (SEO ID NO 1). Hervorgehoben darin sind die verwendeten Oligoribonukleotide, die dadurch gekennzeichnet sind, dass sie zu einem solchen Abschnitt des Zielgens und insbesondere der entsprechenden doppelsträngigen cDNA homolog sind, dessen sense-Strang 5'-seitig durch zwei Adenosinreste (AA) und mit einem Abstand von 19 Basen 3'-seitig durch zwei Thymidinreste (tt), einen Guanosin- und einen Cytosinrest (gc) oder einen Thymidin- und einen Cytidinrest (tc) begrenzt wird.

### Beispiel 14: klares Light-Shampoo mit Volumeneffekt

**Tabelle 10:**

| | |
|---|---|
| Natriumlaurethsulfat | 9,00 |
| Cocoamidopropylbetain | 2,50 |
| anti-TGFbeta-1-dsRNA (dsRNA aus SEQ ID NOs 6 und 7, s. Figur 2) | 0,10 |
| Konservierungsmittel, Parfüm, Verdicker, pH-Einstellung und Lösungsvermittler | q.s. |
| Wasser | ad 100,0 |
| Der pH-Wert wird auf 5,5 eingestellt. | |

### Beispiel 15: Haarkuren

Durch Mischen der in der Tabelle angegebenen Komponenten wurden Haarkuren hergestellt. Als Oligoribonukleotid wurde dsRNA verwendet, die durch Hybridisieren der SEQ ID No 6 und der SEQ ID No 7 erhalten wurde Die dsRNA inhibiert die Expression des Wachstumsfaktors TGFbeta-1 durch RNA-Interferenz und wird als anti-TGFbeta-1-dsRNA bezeichnet (s. Figur 2). Die beiden Stränge der dsRNA wiesen 3'-ständig jeweils zwei 2'-Desoxythymidinreste auf.

**Tabelle 11:**

| | 1 | 2 | 3 |
|---|---|---|---|
| Hydroxypropylmethylcellulose | 0,5% | 0.5% | 0,5% |
| Cetrimoniumbromid | 1,0 | - | 0,8 |
| Behentrimoniumchlorid | - | 0,7 | 0,3 |
| Glycerin | 3,0 | 3,0 | 3,0 |
| Cetearylalkohol | 2,5 | 2,5 | 2,5 |
| Glycerylstearat | 2,0 | 2,0 | 2,0 |
| Polyquaternium-10 | 0,1 | - | - |
| Guar Hydroxypropyl Trimonium Chlorid | - | 0,2 | - |
| anti-TGFbeta-1-dsRNA (dsRNA aus SEQ ID NOs 6 und 7, s. Figur 2) | 0,1 | 0,1 | 0,1 |
| Konservierungsmittel, Parfüm, pH-Einstellung und Lösungsvermittler | q.s. | q.s. | q.s. |
| Wasser | ad 100 | ad 100 | ad 100 |

### Beispiel 16: Haarspülungen

**Tabelle 12:**

| | 1 | 2 | 3 |
|---|---|---|---|
| Cetrimoniumchlorid | 1,0 | 0,5 | 0,5 |
| Behentrimoniumchlorid | - | 0,2% | 0,3 |
| Glycerin | 3,0 | 3,0 | 3,0 |
| Hydroxyethylcellulose | 0,2 | 0,2 | 0,2 |
| Polyquaternium-10 | 0,1 | - | - |
| Guar Hydroxypropyl Trimonium Chlorid | - | 0,2 | - |
| anti-TGFbeta-1-dsRNA (dsRNA aus SEC ID NOs 6 und 7, s. Figur 2) | 0,1 | 0,1 | 0,1 |
| Konservierungsmittel, Parfüm, pH-Einstellung und Lösungsvermittler | q.s. | q.s. | q.s. |
| Wasser | ad 100 | ad 100 | ad 100 |

### Beispiel 17: Leave-on Conditioner

**Tabelle 13:**

| | 1 | 2 | 3 | 4 |
|---|---|---|---|---|
| Cetylalcohol | 1,5 | 1,8 | 2,0 | - |
| Cetrimoniumchtorid | 0,3 | 0,1 | 0,5 | 0,5 |
| Behentrimoniumchlorid | - | 0,2 | - | - |
| Benzophenone- | 0,05 | 0,03 | - | 0,1 |
| 4 | | | | |
| PVPNA Copolymer | 0,4 | | - | - |
| Polyquatemium-37 | - | - | - | 1,0 |
| Polyquatemium-4 | - | - | - | 0,2 |
| Polyquatemium-10 | - | - | 0,5 | - |
| Panthenol | 0,1 | - | 0,2 | 0,1 |
| Hydroxyethylcellulose | - | - | - | 0,3 |
| Acrylates/C10-30 Alkyl Acrylates Crosspolymer | 0,5 | 0,3 | 0,2 | - |
| C12-13 Alkyl Lactate | 2,0 | 1,0 | 1,5 | 1,0 |
| Laureth-4 | - | - | - | 0,5 |
| Aluminium Starch Octenylsuccinate | - | - | - | 1,0 |
| Dicaprylyl Carbonate | - | - | - | 1,0 |
| anti-TGFbeta-1-dsRNA (dsRNA aus SEG ID NOs 6 und 7, s. Figur 2) | 0,1 | 0,1 | 0,1 | 0,1 |
| Konservierungsmittel, Parfüm, pH-Einstellung und Lösungsvermittler | q.s. | q.s. | q.s. | q.s. |
| Wasser | ad 100 | ad 100 | ad 100 | ad 100 |

### Beispiel 18: Spray-Conditioner

**Tabelle 14:**

| | 1 | 2 | 3 | 4 |
|---|---|---|---|---|
| Cetrimoniumchlorid | 0,2 | 0,1 | 0,8 | 0,5 |
| Behentrimoniumchtorid | - | 0,2 | 0,3 | - |
| Benzophenone-4 | 0,05 | 0,03 | - | - |
| PVPNA Copolymer | - | 0,7 | - | - |
| Polyquatemium-10 | 0,1 | - | - | - |
| Polyquatemium-4 | 0,2 | - | - | - |
| Propylene Glycol | - | - | - | 3,0 |
| Polyquatemium-11 | - | - | 0,2 | - |
| Panthenol | 0,1 | - | 0,2 | 0,1 |
| Glyceryl Isostearate | - | - | - | 0,4 |
| Isoceteth-20 | - | - | - | 0,8 |
| Dicaprylyl Carbonate | - | - | - | 0,5 |
| anti-TGFbeta-1-dsRNA (dsRNA aus SEQ ID NOs 6 und 7, s. Figur 2) | 0,1 | 0,1 | 0,1 | 0,1 |
| Konservierungsmittel, Parfüm, pH-Einstellung und Lösungsvermittler | q.s. | q.s. | q.s. | q.s. |
| Wasser | ad 100 | ad 100 | ad 100 | ad 100 |

### Beispiel 19: Haarwässer

**Tabelle 15:**

| | 1 | 2 | 3 | 4 |
|---|---|---|---|---|
| Ethanol | 30,0 | 50,0 | - | - |
| Isopropanol | - | - | 40,0 | 30,0 |
| Panthenol | 0,2 | 0,1 | 0,2 | 0,2 |
| Menthol | 0,1 | - | 0,05 | 0,05 |
| Tocopheryl Acetate | 0,2 | 0,2 | - | 0,1 |
| C12-13 Alkyl Lactate | 0,2 | 0,1 | 0,2 | - |
| anti-TGFbeta-1-dsRNA (dsRNA aus SEQ ID NOs 6 und 7, s. Figur 2) | 0,1 | 0,1 | 0,1 | 0,1 |
| Parfüm, pH-Einstellung und Lösungsvermittler | q.s. | q.s. | q.s. | q.s. |
| Wasser | ad 100 | ad 100 | ad 100 | ad 100 |

## Patentansprüche

1. Oligoribonukleotide, die den Abbau der mRNA von Strukturen induzieren, die in stimulierender Art und Weise auf den Haarzyklus Einfluss nehmen, wobei die Strukturen den Transfoming Growth Faktor beta-Superfamilie (TGFbeta) darstellen und die Oligonukleotide gewählt werden unter SEQ ID 6, 7, 15, 16, 18 oder 19.

2. Oligoribonukleotide nach dem vorangehenden Patentanspruch **dadurch gekennzeichnet, dass** es sich um doppelsträngige Oligoribonukleotide handelt, die am 3'-Ende von jedem Strang einen Überhang von 1 bis 6, vorzugsweise 1 oder 2 Nukleotiden aufweisen.

3. Oligoribonukleotide nach einem der vorangehenden Patentansprüche **dadurch gekennzeichnet, dass** sie in Expressionsvektoren integriert werden, Insbesondere solchen, die eine Expression der Ollgoribonukleotide in Säugerzellen bewirken.

4. Oligoribonukleotide nach einem der vorangehenden Patentansprüche **dadurch gekennzeichnet, dass** sie auf der Ebene der Zuckerreste, der Nukleobasen, der Phosphatgruppen und/oder des dazwischen befindlichen Skeletts chemisch modifiziert sind.

5. Oligoribonukleotide nach einem der vorangehenden Patentansprüche **dadurch gekennzeichnet, dass** eine oder mehrere Phosphatgruppen durch Phosphothioat-, Methylphosphonat- und/oder Phosphoramidatgruppen ausgetauscht sind.

6. Oligoribonukleotide nach einem der vorangehenden Patentansprüche **gekennzeichnet durch** den Austausch einer oder mehrerer Ribosereste des Oligoribonukleotids **durch** Morpholinringe (Morpholin-Oligoribonukleotide) oder **durch** Aminosäuren (Peptid-Oligoribonukleotide).

7. Oligoribonukleotide nach einem der vorangehenden Patentansprüche **dadurch gekennzeichnet, dass** deren Ribosereste durch Amino-, wie NH2, Fluor, Alkyl oder O-Alkylreste, wie OCH3, modifiziert sind.

8. Oligoribonukleotide nach einem der vorangehenden Patentansprüche **dadurch gekennzeichnet, dass** sie α-Nukleoside enthalten.

9. Oligoribonukleotide nach einem der vorangehenden Patentansprüche **dadurch gekennzeichnet, dass** sie in verkapselter Form, beispielsweise verkapselt in Liposomen oder durch die Zugabe von Cyclodextrinen stabilisiert verwendet werden.

10. Nicht-therapeutische Verwendung von Oligoribonukleiotiden nach einem der vorangehenden Ansprüche in Zubereitungen zur Behandlung und Prophylaxe alters- und umweltbedingter
degenerativer und defizitärer Erscheinungen der Haarfollikel, der Haut und von Hautanhanggebilden, wie Drüsen.

11. Nicht-therapeutische Verwendung von Zubereitungen nach einem der vorangehenden Ansprüche zum Schutz vor Haarausfall, Haarfollikelminiaturisierung, dünner werdendem Haar, Depigmentierungserscheinungen des Haares, dystrophischen Veränderungen der Haarwurzel insbesondere der Haare auf dem Kopf.

## Claims

1. Oligoribonucleotides which induce the degradation of the mRNA of structures which in a stimulatory manner affect the hair cycle, where the structures are the Transforming Growth Factor beta-superfamily (TGFbeta) and where the oligoribonucleotides are selected among SEQ ID 6, 7, 15, 16, 18 or 19.

2. Oligoribonucleotides according to the preceding claim, **characterized in that** they are double-stranded oligoribonucleotides which have an overhang of from 1 to 6, preferably 1 or 2, nucleotides at the 3' end of each strand.

3. Oligoribonucleotides according to any of the preceding claims, **characterized in that** they are integrated into expression vectors, in particular those which bring about an expression of the oligoribonucleotides in mammalian cells.

4. Oligoribonucleotides according to any of the preceding claims, **characterized in that** they are chemically modified at the level of the sugar residues, of the nucleobases, of the phosphate groups and/or of the backbone located between them.

5. Oligoribonucleotides according to any of the preceding claims, **characterized in that** one or more phosphate groups are exchanged for phosphothioate groups, methylphosphonate groups and/or phosphoramidate groups.

6. Oligoribonucleotides according to any of the preceding claims, **characterized in that** one or more ribose residues of the oligoribonucleotide is/are exchanged for morpholin rings (morpholin oligoribonucleotides) or by amino acids (peptideoligoribonucleotides).

7. Oligoribonucleotides according to any of the preceding claims, **characterized in that** the ribose residues thereof are modified by amino residues, such as NH2, fluorine residues, alkyl residues or O-alkyl residues, such as OCH3.

8. Oligoribonucleotides according to any of the preceding claims, **characterized in that** they comprise α-nucleosides.

9. Oligoribonucleotides according to any of the preceding claims, **characterized in that** they are used in encapsulated form, for example encapsulated in liposomes or stabilized by the addition of cyclodextrins.

10. Nontherapeutic use of oligoribonucleotides according to any of the preceding claims in preparations for the treatment and prophylaxis of age- and environment-associated degenerative and deficient symptoms of the hair follicles, of the skin and of cutaneous appendages such as glands.

11. Nontherapeutic use of preparations according to any of the preceding claims for the protection against hair loss, hair follicle miniaturization, thinning hair, depigmentation symptoms of the hair, dystrophic changes of the hair root, in particular of the hair of the head.

## Revendications

1. Oligoribonucléotides qui induisent la dégradation de l'ARNm de structures qui, d'une manière stimulante, influent sur le cycle capillaire, les structures représentant la superfamille Transforming Growth Factor beta (facteur de croissance transformant bêta) (TGFbeta), et les oligoribonucléotides étant choisis parmi SEQ ID N°6, 7, 15, 16, 18 ou 19.

2. Oligoribonucléotides selon la revendication précédente, **caractérisés en ce qu'**il s'agit d'oligoribonucléotides double brin, qui à l'extrémité 3' de chaque brin comportent une extrémité sortante de 1 à 6, de préférence de 1 ou 2 nucléotides.

3. Oligoribonucléotides selon l'une des revendications précédentes, **caractérisés en ce qu'**ils sont intégrés dans des vecteurs d'expression, en particulier ceux qui provoquent une expression des oligoribonucléotides dans des cellules de mammifères.

4. Oligoribonucléotides selon l'une des revendications précédentes, **caractérisés en ce qu'**ils sont chimiquement modifiés au niveau des résidus de sucre, des nucléobases, des groupes phosphate et/ou du squelette situé entre eux.

5. Oligoribonucléotides selon l'une des revendications précédentes, **caractérisés en ce qu'**un ou plusieurs groupes phosphate sont remplacés par des groupes phosphothioate, méthylphosphonate et/ou phosphoramidate.

6. Oligoribonucléotides selon l'une des revendications précédentes, **caractérisés par** le remplacement d'un ou plusieurs résidus de ribose de l'oligoribonucléotide par des noyaux morpholine (morpholino-oligoribonucléotides) ou par des acides aminés (peptido-oligoribonucléotides).

7. Oligoribonucléotides selon l'une des revendications précédentes, **caractérisés en ce que** leurs résidus de ribose sont modifiés par des résidus amino, tels que NH₂, fluoro, alkyle ou O-alkyle, tels que OCH₃.

8. Oligoribonucléotides selon l'une des revendications précédentes, **caractérisés en ce qu'**ils contiennent des α-nucléosides.

9. Oligoribonucléotides selon l'une des revendications précédentes, **caractérisés en ce qu'**ils sont stabilisés sous forme encapsulée, par exemple encapsulés dans des liposomes, ou par addition de cyclodextrines.

10. Utilisation non thérapeutique d'oligoribonucléotides selon l'une des revendications précédentes dans des préparations destinées au traitement et à la prophylaxie de phénomènes dégénératifs et déficitaires, dus à l'âge et à l'environnement, des follicules pileux, de la peau et des appendices cutanés, tels que les glandes.

11. Utilisation non thérapeutique de préparations selon l'une des revendications précédentes pour la protection contre la chute des cheveux, la miniaturisation des follicules pileux, l'amincissement des poils, les phénomènes de dépigmentation des poils, les modifications dystrophiques des racines des poils, en particulier des cheveux sur la tête.
